# EUROPÄISCHE PATENTSCHRIFT

(11) **EP 3 744 265 B1**
(45) Veröffentlichungstag und Bekanntmachung des Hinweises auf die Patenterteilung: **07.07.2021**
(21) Anmeldenummer: 19000261.8
(22) Anmeldetag: 28.05.2019
(51) Int. Cl.: A61B 17/221, A61B 18/26

(54) **MEDIZINISCHES INSTRUMENT UND VERFAHREN ZUR HERSTELLUNG DESSELBEN**
MEDICAL INSTRUMENT AND METHOD FOR ITS PRODUCTION
INSTRUMENT MÉDICAL ET SON PROCÉDÉ DE FABRICATION

(43) Veröffentlichungstag der Anmeldung: 02.12.2020
(73) Patentinhaber: Endosmart Gesellschaft für Medizintechnik mbH, 76297 Stutensee (DE)
(72) Erfinder: VOGEL, Bernd, Dr., 76199 Karlsruhe (DE)
(74) Vertreter: Mehl-Mikus, Claudia

(56) Entgegenhaltungen:
- EP-A1- 1 018 953
- DE-A1- 3 633 527
- US-A1- 2002 026 203
- US-A1- 2002 068 943
- US-A1- 2019 117 309
- US-B1- 6 264 664

## Beschreibung

Die Erfindung betrifft ein medizinisches Instrument mit einem Werkzeug, das insbesondere ein Steinfangkörbchen ist, und mit einem Lichtwellenleiter wie einer Laserfaser sowie ein Herstellungsverfahren für dieses medizinische Instrument.

Aus dem Stand der Technik ist bekannt, zur Zertrümmerung von Konkrementen in Hohlorganen, wie beispielsweise Gallengang- oder Nierensteinen, Laserlithotripter mit einer Fangeinrichtung wie einem Steinfangkörbchen einzusetzen, mit dem ein Konkrement gehalten wird, während es mit dem Lithotripter mittels Laserlichtimpulsen zerkleinert bzw. zerstört wird. Dabei können Steinfangkörbchen und Laserlithotripter in den Arbeitskanälen zweier separater endoskopischer Instrumente geführt werden, wodurch vorteilhaft eine ausreichende Spülung des Arbeitsfelds sichergestellt ist, nachteilig aber die exakte Positionierung von Körbchen und Lichtleiterspitze zueinander schwierig ist, um ein mit dem Körbchen gefangenes Konkrement zerstören zu können, ohne die Drahtschlingen des Steinfangkörbchens, auch Körbchenlitzen genannt, oder auch das umgebende Gewebe durch Laserimpulse zu beschädigen.

US 2002/0068943 A1 offenbart ein Fanginstrument, das nicht mit einem Laserlithotripter kombiniert geführt wird, wobei die Drahtschlingen aus einem gegenüber laserinduzierten Beschädigungen resistenten Material sind und innerhalb einer äußeren Hülse über die Befestigung an einem inneren Zugdraht aus- und eingefahren werden können.

Auch EP 1 018 953 A1 zeigt ein solches Fanginstrument mit einem aus mehreren Drahtabschnitten gebildeten Körbchen, deren Enden durch einen äußeren hohlen Schaft bis zum Handstück verlaufen, an einem oder mehreren innerhalb des äußeren Schafts verschiebbaren inneren Führungselementen oder direkt am Schaft befestigt sind.

Um eine gezielte sichere Applikation der Laserenergie auf das Konkrement sicherzustellen, offenbart DE 36 33 527 A1 ein Instrument, bei dem das Steinfangkörbchen koaxial zu einem axial verschiebbaren Lichtleiter ausgefahren werden kann, um mit dem Körbchen Steine fixieren und den Lichtleiter zentral auf den Stein aufsetzen zu können, um diesen zu zertrümmern, ohne umliegendes Gewebe zu tangieren. Das Instrument weist einen äußeren Schlauch auf, in dem der Laserlichtleiter, ein Zugdraht für das Körbchen und die daran befestigten Körbchenlitzen geführt werden. Zur koaxialen Anordnung des Laserlichtleiters mit dem Steinfangkörbchen wird der Laserlichtleiter, der von einem inneren Schlauch aus inertem Material umgeben ist, am distalen Ende in einer (Metall-) Führungshülse mit einer Bohrung geführt. Diese Führungshülse ist in dem äußeren Schlauch angeordnet und weist an der äußeren Manteloberfläche Längsnuten auf, in denen die Körbchenlitzen geführt werden. Durch den Durchmesser der Laserfaser vorgegeben, bauen sich Durchmesser des inneren Schlauchs, der Führungshülse und des äußeren Schlauchs bis zu einem Außendurchmesser von bis zu 3,2 mm auf, was sich auf die Flexibilität des Instruments auswirkt und auch das Spülen des Arbeitsgebiets mit diesem Instrument nur beschränkt zulässt, da die Führungshülse, in deren Bohrung der Lichtleiter mit dem umgebenden inneren Schlauch aufgenommen ist, die Zufuhr von Spülwasser im Wesentlichen nur durch die Längsnuten ermöglicht.

Auf diesem Gebiet offenbaren außerdem Wilson et al. in "A Miniaturized, 1.9F Integrated Optical Fiber and Stone Basket for Use in Thulium Fiber Laser Lithotripsy", Journal of Endourology, Vol. 29, No. 10 Oktober 2015, S. 1110-1114, die Verwendung eines Thuliumlasers unter Nutzung einer Laserfaser mit geringerem Außendurchmesser von 140 µm in einem miniaturisierten Instrument, in dem Laserfaser und Steinfangkörbchen kombiniert genutzt werden. Die Laserfaser wird neben einem 1.3F (433 µm) Steinfangkörbchen und dessen Handhabungsdraht in einer 1.9F (633 µm) Einführhülle angeordnet, sodass Laserfaser und Steinfangkörbchen zwar zusammen, aber nicht koaxial vorliegen.

Ferner ist in US 6,264,664 B1 ein Instrument mit einem Körbchen und einem Lichtleiter offenbart, wobei der Lichtleiter in einem inneren Schlauch vorliegt, an dem zugleich das proximale Ende des Körbchens befestigt ist. Der innere Schlauch liegt in einer äußeren Hülse vor und kann relativ gegenüber diesem bewegt werden, damit sich das Körbchen mit Formgedächtnis beim Austritt aus dem äußeren Schlauch öffnet.

Ausgehend von diesem Stand der Technik ist es Aufgabe der vorliegenden Erfindung, ein Steinfangkörbchen mit integriertem Lichtwellenleiter dahingehend zu verbessern, dass laserinduzierte Schäden am Steinfangkörbchen und Schäden an dem Lichtwellenleiter verringert werden.

Diese Aufgabe wird durch ein medizinisches Instrument mit den Merkmalen des Anspruchs 1 gelöst.

Die weitere Aufgabe, ein solches medizinisches Instrument herzustellen, wird durch das Verfahren mit den Merkmalen des unabhängigen Anspruchs 14 gelöst.

Weiterbildungen sind in den jeweiligen Unteransprüchen ausgeführt.

Eine erste Ausführungsform des erfindungsgemäßen medizinischen Instruments weist ein Werkzeug, eine Katheteranordnung und einen Griff auf, mit dem das Werkzeug, das ein Drahtgebilde mit zumindest zwei Drahtabschnitten ist, betätigt werden kann. Der Griff ist an einem distalen Ende der Katheteranordnung angeordnet. Die Katheteranordnung ist dabei aus einem äußeren Schlauch und einem koaxial dazu angeordneten inneren Schlauch gebildet. Das medizinische Instrument weist ferner einen Lichtwellenleiter auf, der sich durch den inneren Schlauch erstreckt und dessen Austrittsende koaxial in einen von dem Werkzeug begrenzten Raum mündet. Erfindungsgemäß ist dabei zumindest ein erster Drahtabschnitt des Drahtgebildes mit einem proximalen Ende an dem distalen Ende des inneren Schlauchs und zumindest ein zweiter Drahtabschnitt des Drahtgebildes mit einem proximalen Ende an dem distalen Ende des äußeren Schlauchs befestigt. Hierbei sind der äußere Schlauch und der innere Schlauch relativ zueinander beweglich, sodass das Werkzeug zum Öffnen und Schließen durch die Relativbewegung zwischen dem äußeren Schlauch und dem inneren Schlauch betätigt werden kann, wobei die Drahtenden gegeneinander verschoben und das Drahtgebilde damit verformt werden. Somit benötigt das medizinische Instrument vorteilhaft zur Betätigung des Werkzeugs keinen Führungsdraht.

Das Werkzeug kann gemäß einer erfindungsgemäßen Ausführungsform ein Greif- oder Fangwerkzeug wie ein Steinfangkörbchen sein.

Indem das medizinische Instrument zur Betätigung des Werkzeugs keinen Führungsdraht benötigt, unterscheidet es sich vorteilhaft von den herkömmlichen Instrumenten, bei denen sich ein Führungsdraht von dem Werkzeug (einem Steinfangkörbchen oder einem anderen Greifwerkzeug) durch die Schlauchanordnung bis zum Griff erstreckt.

Die Katheteranordnung aus dem relativ zu dem inneren Schlauch beweglichen äußeren Schlauch übernimmt die Funktion des Führungsdrahts und gestattet mit der Anordnung des Lichtwellenleiters im inneren Schlauch, der direkt im äußeren Schlauch geführt wird, eine koaxiale Anordnung des Lichtwellenleiteraustritts in dem vom Werkzeug begrenzten Raum, ohne dass weiter Mittel oder Maßnahmen zum Schutz des Lichtwellenleiters gegenüber einem Führungsdraht erforderlich sind. Dadurch kann der Durchmesser der Katheteranordnung mit dem koaxialen Lichtwellenleiter vorteilhaft deutlich reduziert werden, was die Flexibilität des Instruments erhöht. Dabei sind die Durchmesser des Lichtwellenleiters, des inneren und äußeren Schlauchs aufeinander abgestimmt, sodass kein oder kaum Spiel zwischen dem Lichtwellenleiter, inneren und äußeren Schlauch besteht, d. h. der Innendurchmesser des inneren Schlauch entspricht dem Durchmesser des Lichtwellenleiters und der Innendurchmesser des äußeren Schlauchs dem Außendurchmesser des inneren Schlauchs jeweils innerhalb eines Toleranzbereiches, der das ineinander Führen und Verschieben zulässt.

Sowohl die Laserfaser also auch der Innendurchmessers des Schlauches haben Fertigungstoleranzen. Das Spaltmaß zwischen diesen beiden Komponenten muss, wie der Fachmann weiß, entsprechend ausgelegt sein, sodass die Laserfaser sich auch noch im schlechtesten Falle, bei größtem möglichen Durchmesser der Laserfaser und kleinstem möglichen Innendurchmesser des Schlauchs, verschieben lässt. Basierend auf diesem Gedanken kann man von einem Spaltmaß in der Größenanordnung von 10 % des Laserfaserdurchmessers ausgehen.

Zudem ermöglicht die koaxiale Anordnung des Lichtwellenleiteraustritts in dem vom Werkzeug begrenzten Raum einen Lichtstrahl zentral auf einen mit dem Werkzeug erfassten Gegenstand zu richten, z. B. einen mit einem Steinfangkörbchen gefangenen Stein mit einem zentral darauf gerichteten Laserstrahl zu zertrümmern, ohne umliegendes Gewebe zu tangieren. Dieses Risiko besteht, wenn Steinfangkörbchen und Lichtleitfaser nebeneinander angeordnet sind.

Für eine weitere Verbesserung der Flexibilität und des Spülflusses sorgt in einer weiteren Ausführungsform des medizinischen Instruments die Ausbildung des Lichtwellenleiters als eine Laserfaser, die zur Leitung von Laserstrahlung eines Thuliumlasers ausgebildet ist, der die Nutzung einer Laserfaser mit deutlich reduziertem Durchmesser von 200 µm und kleiner ermöglicht. Zur weiteren Querschnittsreduktion ist hierbei vorgesehen, dass die verwendete Laserfaser kein Buffering (Schutzbeschichtung) sondern nur den Kern, der aus Material mit höherem Brechungsindex besteht, und das umgebende Cladding bzw. Mantelglas aufweist, das aus einem dielektrischen Material mit niedrigerem Brechungsindex besteht. Mit der Einsparung des Bufferings wird die Steifigkeit reduziert und damit die Flexibilität des Instruments weiter verbessert. Die Aufgaben des Bufferings, wie z. B. Biegeschutz, werden durch den inneren Schlauch, der die Laserfaser umgibt, übernommen.

Die vorliegend verwendeten Begriffe "distal" und "proximal" zur Beschreibung von Positionsangaben am Instrument sind in Bezug auf den Anwender des Instruments zu verstehen, d. h. "distal" ist ein weiter vom Anwender, der das Instrument am Griff bedient, beabstandeter Instrumententeil, wohingegen "proximal" demgegenüber einen näher beim Anwender zu liegen kommenden Instrumententeil meint. Daher werden der Endbereich des Instruments mit der Arbeitsspitze aus Werkzeug und Lichtleiteraustrittsende als "distal" und der Endbereich des Instruments mit dem Griff bzw. dessen Endabschnitt, an dem der Lichtwellenleiter zur Verbindung mit einer Lichtquelle ausgekoppelt wird, als "proximal" bezeichnet. Abgeleitet davon gibt "distal" Stellen, Abschnitte oder Richtungen an, die an der bzw. näher zur Arbeitsspitze liegen, während "proximal" Stellen, Abschnitte oder Richtungen beschreibt, die am bzw. näher zum Auskoppelgriffende liegen.

In einer weiteren Ausführungsform kann der Griff des medizinischen Instruments einen Griffkörper und zumindest ein Betätigungselement zur Betätigung des Werkzeugs, das insbesondere ein Steinfangkörbchen sein kann, aufweisen. Dazu ist ein proximaler Endabschnitt des äußeren Schlauchs mit dem Betätigungselement verbunden, während ein proximaler Endabschnitt des inneren Schlauchs, der im Griff über das proximale Ende des äußeren Schlauchs hinausragt, im Griff ortsfest gelagert ist, sodass sich der äußere Schlauch zur Betätigung des Werkzeugs mittels des Betätigungselements gegenüber dem inneren Schlauch axial verschieben lässt. Alternativ ist aber auch denkbar, dass umgekehrt ein proximaler Endabschnitt des äußeren Schlauchs im Griff gelagert ist, und ein proximaler Endabschnitt des inneren Schlauchs, der im Griff über das proximale Ende des äußeren Schlauchs hinausragt, mit dem Betätigungselement verbunden ist, sodass der innere Schlauch zur Betätigung des Werkzeugs mittels des Betätigungselements gegenüber dem äußeren Schlauch axial verschiebbar ist.

Beispielsweise kann das Betätigungselement in einer Ausführung ein Griffschieber sein, der zur Bewegung des äußeren (bzw. inneren) Schlauchs in axialer Richtung gegen den Griffkörper verschoben werden kann. Ein dazu alternatives Betätigungselement kann beispielsweise ein Stellrad sein.

Alternativ oder zusätzlich kann in der Ausführungsform, bei der das Betätigungselement mit dem proximalen Endabschnitt des äußeren Schlauchs verbunden ist, diese Verbindung über eine Gleithülse bereitgestellt werden, die mit dem Betätigungselement verbunden ist und in der eine Verbindungshülse gelagert ist, in der der äußere Schlauch befestigt ist. Durch eine solche Verbindungshülse erstreckt sich die Verbindungsfläche mit dem äußeren Schlauch über einen längeren axialen Abschnitt, wodurch das Verschieben des äußeren Schlauchs gegenüber dem inneren Schlauch unterstützt wird. Grundsätzlich kann aber das Betätigungselement auch direkt mit dem proximalen Endabschnitt des äußeren Schlauchs verbunden, bzw. daran befestigt sein.

In einer weiteren Ausführungsform kann vorgesehen sein, dass die Lagerung des proximalen Endabschnitts des inneren Schlauchs im Griff durch eine Fixierhülse bereitgestellt wird, in der der innere Schlauch befestigt ist und die mit ihrem distalen Ende an einer Lagervorrichtung anliegt, die zwischen dem proximalen Ende des äußeren Schlauchs und der Fixierhülse im Griffkörper oder in einem Griffkörperaufsatz vorgesehen ist, der sich an ein proximales Ende des Griffkörpers anschließt. Mit der gegen diese Lagervorrichtung anliegenden Fixierhülse wird beim Verschieben des äußeren Schlauchs in distaler Richtung eine Mitbewegung des inneren Schlauchs verhindert, sodass die Drahtenden des Werkzeugs gegeneinander verschoben werden können.

Eine solche Lagervorrichtung kann einfach ein im Griffkörper oder in einem Griffkörperaufsatz ausgebildeter Ringabsatz sein, an dem die Fixierhülse anliegt. In einer bevorzugten Ausführungsform ist jedoch vorgesehen, dass die Lagervorrichtung gleichzeitig für einen Überlastungsschutz sorgt und durch eine Feder (Schraubenfeder) bereitgestellt wird, an deren proximalen Ende die Fixierhülse anliegt, wobei sich das distale Ende der Feder an einem Ringabsatz abstützt, der im Griffkörper vorliegt. Durch diese gefederte Lagerung wird die Fixierhülse durch die Eigen- bzw. eine Vorspannung der Feder beim Verschieben des äußeren Schlauchs zur Betätigung des Werkzeugs bis zu einer vorbestimmten Krafteinwirkung in ihrer Position gehalten. Wird diese vorbestimmte Kraft überschritten, wenn z. B. bei einem mit einem Steinfangkörbchen eingefangenen großen Stein versucht wird, das Steinfangkörbchen zu schließen, sodass die auf die Drahtschlingen wirkenden Zugkräfte drohen, zu groß werden, wird die Fixierhülse und damit der innere Schlauch gegen die Feder in distale Richtung bewegt, um eine Beschädigung der Drahtschlingen zu verhindern.

Der Ringabsatz, an dem sich das distale Ende der Feder im Griffkörper abstützt, kann dabei in einer bevorzugten Ausführungsform durch ein proximales Ende einer Führungshülse bereitgestellt werden, die im Griffkörper angeordnet ist und für eine Führung der Gleithülse sorgt, die mit dem Griffschieber verbunden ist. Damit wird der äußere Schlauch, der über die verbundene Verbindungshülse in der Gleithülse gelagert ist, beim Verschieben sicher in axialer Richtung geführt.

Ferner ist in einer weiteren Ausführungsform vorgesehen, dass der Lichtwellenleiter über das proximale Ende des inneren Schlauchs hinausragt und sich durch den proximalen axialen Endabschnitt des Griffs aus dem Griff zur Verbindung mit einer Laserlichtquelle, vorzugsweise einem Thuliumlaser erstreckt. Der proximale axiale Endabschnitt kann dabei in einer bevorzugten Ausführung eine ringförmige Dichtlippe aufweisen, die abdichtend um den Lichtwellenleiter anliegt. Besonders bevorzugt kann diese ringförmige Dichtlippe radial verstellbar sein bzw. einen wie bei einer Blende radial verstellbaren Öffnungsquerschnitt aufweisen, sodass sich die auf den Umfang der Laserfaser wirkende Dichtkraft einstellen lässt. Derartige axiale Endabschnitte mit verstellbarer Dichtlippe, die über einen drehbaren Gehäuseabschnitt betätigt wird, sind als Tuohy-Borst-Adapter erhältlich.

Vorzugsweise kann diese Dichtkraft so eingestellt sein, dass gemäß einer weiteren Ausführungsform eine axiale Verschiebung des Lichtwellenleiters in dem inneren Schlauch möglich ist, ohne die Laserfaser zu beschädigen. Alternativ kann die Dichtung zum Verschieben der Laserfaser etwas gelöst, d. h. der Querschnitt der Dichtung geöffnet und nach erfolgter Positionierung wieder geschlossen werden. In einer bevorzugten Ausführungsform des axial verschiebbar in dem inneren Schlauch angeordneten Lichtwellenleiters kann der Griff ein zweites Betätigungselement aufweisen, um das Verschieben des Lichtwellenleiters einfach und komfortabel bereitzustellen. Dieses zweite Betätigungselement kann eine Verschiebevorrichtung umfassen oder damit verbunden sein, die mit dem Lichtwellenleiter in Wirkverbindung steht und die eine axiale Verschiebung des Lichtwellenleiters bereitstellt. Auch hier sind als geeignete Betätigungselemente Griffschieber oder Stellrädchen denkbar. Vorzugsweise kann die zweite Betätigungsvorrichtung zum Verschieben des Lichtwellenleiters im Bereich des proximalen axialen Endabschnitts des Griffkörpers vorgesehen sein, etwa zwischen dem Auskoppelende und der Dichtlippe oder zwischen der Dichtlippe und der Fixierhülse.

Grundsätzlich kann der Griff ferner eine Einführhilfe für den Lichtwellenleiter in den inneren Schlauch aufweisen, die an ihrem proximalen Ende eine sich auf den Innenquerschnitt des inneren Schlauchs verjüngende, z. B. trichterartige Einführöffnung aufweist, die das Einführen des Lichtwellenleiters in den inneren Schlauch bei der Montage des medizinischen Instruments vereinfacht. Eine solche Einführhilfe kann sinnvoller Weise am proximalen Griffende bzw. zumindest am proximalen Ende der Fixierhülse oder einer anderen Einrichtung, die den inneren Schlauch fixiert, vorgesehen sein.

Bei dem als Drahtgebilde ausgebildeten Werkzeug eines erfindungsgemäßen medizinischen Instruments kann es sich um ein Greifwerkzeug aus einer einzigen Drahtschlinge handeln, die aus einem ersten und damit verbundenen zweiten Drahtabschnitt gebildet ist, wobei ein proximales Ende der Drahtschlinge mit dem inneren Schlauch und das andere proximale Ende der Drahtschlinge mit dem äußeren Schlauch verbunden ist.

Eine bevorzugte Ausführungsformen des medizinischen Instruments sieht als Drahtgebilde-Werkzeug ein Steinfangkörbchen vor, mit dem Konkremente wie Nieren- oder Gallensteine eingefangen werden können, die durch Laserstrahlen aus dem koaxial angeordneten Lichtwellenleiter zertrümmert werden sollen. Dabei kann ein erfindungsgemäßes medizinisches Instrument mit unterschiedlichen Steinfangkörbchen ausgestattet sein. Das Steinfangkörbchen kann aus zwei oder drei Drahtschlingen gebildet sein, die jeweils aus einem ersten Drahtabschnitt und einem damit verbundenen zweiten Drahtabschnitt gebildet sind. Dabei kann jede Drahtschlinge mit einem ersten proximalen Ende an dem inneren Schlauch und mit einem zweiten proximalen Ende an dem äußeren Schlauch befestigt sein. So kann das Steinfangkörbchen in einer Ausführungsform ein geschlossenes Steinfangkörbchen sein, das aus zwei Drahtschlingen ausgebildet ist, von denen jede Drahtschlinge einstückig aus einem ersten und einem damit verbundenen zweiten Drahtabschnitt besteht. Die Drahtschlingen können sich in einem Bereich, der dem Austrittsende des Lichtwellenleiters gegenüberliegt, kreuzen oder tangieren, um so das Steinfangkörbchen zu schließen. Die Drahtschlingen können vorzugsweise in diesem Kreuzungs- bzw. Berührungspunkt miteinander verbunden sein.

Alternativ kann ein geschlossenes Steinfangkörbchen aus vier separaten Drahtabschnitten gebildet werden, beispielsweise zwei ersten und zwei zweiten Drahtabschnitten, sodass jeweils zwei erste Drahtabschnitte an ihrem proximalen Ende mit dem inneren Schlauch verbunden sind, und jeweils zwei zweite Drahtabschnitte an ihrem proximalen Ende an dem äußeren Schlauch befestigt sind, während die jeweils anderen, distalen Enden der Drahtabschnitte in einem dem Kreuzungs- bzw. Berührungspunkt entsprechenden Bereich, der dem Austrittsende des Lichtwellenleiters gegenüberliegt, miteinander, ggf. unter Verwendung einer Kappenförmigen Spitze oder einer Verbindungsfläche verbunden sind. Dabei können die zwei ersten und die zwei zweiten Drahtabschnitte jeweils nebeneinander oder vorzugsweise symmetrisch gegenüberliegend angeordnet sein. Gegebenenfalls ist es aber auch denkbar, dass ein geschlossenes Steinfangkörbchen unsymmetrisch aus einem ersten und drei zweiten Drahtabschnitten oder drei ersten und einem zweiten Drahtabschnitt gebildet werden kann, wobei der erste oder die drei ersten Drahtabschnitte an ihrem proximalen Ende mit dem inneren Schlauch verbunden sind, und die entsprechend drei zweiten Drahtabschnitte oder der eine zweite Drahtabschnitt an dem proximalen Ende mit dem äußeren Schlauch verbunden sind/ist.

In einer weiteren alternativen Ausführungsform können die vier Drahtabschnitte, die ein geschlossenes Steinfangkörbchen bilden, einstückig durch einen einzigen Draht dargestellt werden, der an drei gleichmäßig distanzierten Biegestellen in alternierender Richtung spitzwinklig gebogen ist, sodass der einzige Draht in vier zusammenhängende Drahtabschnitte unterteilt wird, von denen jeweils zwei benachbarte Drahtabschnitte jeweils eine Drahtschlinge bilden. Dabei können die erste und dritte Biegestelle in dem Bereich liegen, der dem Austrittsende des Lichtwellenleiters gegenüberliegt, und dort verbunden sein, während die freien Drahtenden des einzigen Drahtes beide proximale Enden entweder eines ersten oder zweiten Drahtabschnitts bilden, die an einem von inneren und äußeren Schlauch befestigt sind, und die zweite Biegestelle quasi die entsprechenden proximale Enden entweder eines zweiten oder ersten Drahtabschnitts bilden, die an dem jeweils anderen von inneren und äußeren Schlauch befestigt sind. Es ist umgekehrt aber auch denkbar, dass die erste und dritte Biegestelle proximal angeordnet jeweils an dem inneren und dem äußeren Schlauch befestigt sind, während die freien Enden und die zweite Biegestelle distal in dem Bereich, der dem Austrittsende des Lichtwellenleiters gegenüberliegt, verbunden sind.

In einer besonders bevorzugten Ausführungsform kann das Steinfangkörbchen eines erfindungsgemäßen medizinischen Instruments als ein offenes Steinfangkörbchen ausgebildet sein, das vorzugsweise drei Drahtschlingen jeweils mit einem ersten und einem zweiten Drahtabschnitt aufweist, die umfänglich nebeneinander angeordnet sind, ohne sich zu kreuzen. In einer dabei bevorzugten Ausführungsform kann jeweils ein erster Drahtabschnitt mit dem an dem inneren Schlauch befestigten proximalen Ende jeder Drahtschlinge zusammen mit einem zweiten Drahtabschnitt mit dem an dem äußeren Schlauch befestigten proximalen Ende der jeweils benachbarten Drahtschlinge in einer gemeinsamen Umhüllung geführt werden, mit der das Öffnen und Schließen des Steinfangkörbchens geführt wird. Jede Umhüllung kann eine Hülse mit zwei Durchtrittsöffnungen für den jeweiligen Drahtabschnitt sein. Die Umhüllungen können dabei ebenfalls an dem äußeren Schlauch befestigt sein, beispielsweise mittels eines Schrumpfschlauchabschnitts, oder sie können unbefestigt vorliegen. Die losen Umhüllungen bieten den Vorteil, dass sich das Steinfankörbchen weiter öffnen kann, da die Biegestellen der Drahtschlingen an der Verbindung zu den Schläuchen frei liegen. Durch die Befestigung der Umhüllungen an dem äußeren Schlauch hingegen wird ein Verrutschen der Umhüllungen sicher verhindert, was andernfalls zu einer Behinderung bei der Betätigung des Steinfangkörbchens führen könnte.

Grundsätzlich sind erfindungsgemäß aber auch geschlossene und offene Steinfangkörbchen oder andere Greifwerkzeuge mit einer von zwei oder drei abweichenden Anzahl von Drahtabschnitten denkbar und sollen in entsprechenden Modifikationen der obigen Ausführungsformen umfasst sein.

Die das Drahtgebilde bildenden Drahtabschnitte sind in einer bevorzugten Ausführungsform aus Nitinol und können eine Vorspannung für die geöffnete Position des Werkzeugs bzw. Steinfangkörbchens aufweisen. Zur Verbesserung der Stabilität und der Funktionalität kann/können die Drahtschlinge(n) zumindest teilweise als Flachdraht ausgeführt sein.

Um eine Sterilisation des medizinischen Instruments ohne Verformungsgefahr zu ermöglichen, ist in einer weiteren Ausführungsform vorgesehen, dass der äußere Schlauch aus einem hitzebeständigen Kunststoff gefertigt ist. Ein Beispiel dafür sind Polyimide, die neben ihrer chemischen Beständigkeit eine hohe Hitzebeständigkeit mit Dauereinsatztemperaturen von bis zu 230 °C aufweisen und kurzzeitig bis 400 °C hitzestabil sind.

Der innere Schlauch kann in einer bevorzugten Ausführungsform zum Biege- bzw. Knickschutz des Katheters insgesamt und insbesondere des Lichtwellenleiters eine Faserarmierung aufweisen, die zumindest an der Außenseite mit einer reibungsverringernden Kunststoffbeschichtung (z. B. Polytetrafluorethylen) versehen ist, um eine reibungsarme Relativbewegung zwischen dem inneren und dem äußeren Schlauch zu unterstützen. Die Faserarmierung, die beispielsweise als Geflecht ausgeführt sein kann, kann bevorzugt Metallfasern aufweisen oder daraus bestehen; es sind aber auch Verstärkungsfasern aus anderen Materialien wie Kunststoffe (z. B. Aramid), Carbon oder auch Glasfasern denkbar, da sie im Schlauch eingebettet sind wenn gleich letztere nicht bevorzugt sind, da sie sehr spröde sind.

Abhängig von dem Material des äußeren Schlauchs kann daher die Befestigung der proximalen Enden jedes zweiten Drahtabschnitts an dem äußeren Schlauch durch Kleben (z. B. Epoxy-, Polyurethan- oder Silikonkleber), Einschmelzen oder Schweißen (per Induktion, Vibration oder Ultraschall) erfolgen. Bevorzugt kann alternativ oder zusätzlich dazu ein Schrumpfschlauchabschnitt über einen Verbindungsabschnitt am Ende des äußeren Schlauchs und die proximalen Enden des zumindest einen zweiten Drahtabschnitts angewendet werden.

Die Befestigung der proximalen Enden jedes ersten Drahtabschnitts an dem inneren Schlauch kann in äquivalenter Weise ebenfalls durch Kleben (z. B. Epoxy-, Polyurethan- oder Silikonkleber), Einschmelzen oder Schweißen (per Induktion, Vibration oder Ultraschall) und/oder einen zusätzlichen Schrumpfschlauchabschnitt erfolgen, der sich über einen Verbindungsabschnitt am Ende des inneren Schlauchs und die proximalen Enden jedes ersten Drahtabschnitts erstreckt.

Mit der Faserarmierung des inneren Schlauchs bieten sich aber auch dazu alternative Befestigungsformen der proximalen Enden jedes ersten Drahtabschnitts an dem inneren Schlauch an: So können die Faserenden der Faserarmierung am distalen Ende des inneren Schlauchs oder zumindest ein Teil dieser Faserenden reibschlüssig und/oder stoffschlüssig mit den proximalen Enden jedes ersten Drahtabschnitts verbunden werden. Unter einer reibschlüssigen Verbindung werden hierbei sämtliche Arten der Verknotung, Verflechtung, Verschlingung verstanden, durch die ein ausreichender Reibschluss zwischen den Faserenden und den proximalen Enden jedes ersten Drahtabschnitts erzeugt werden kann. Die stoffschlüssige Verbindung kann durch Verkleben oder Verschweißen erfolgen, wobei es sich vorzugsweise bei den Fasern der Faserarmierung um Metallfasern handeln kann, die besonders bevorzugt aus einem den Drahtabschnitten entsprechenden Material bestehen können.

Wenn die Faserarmierung des inneren Schlauchs Fasern aus einem Material aufweist oder daraus besteht, die dem Metall der Drahtabschnitte entsprechen, z. B. aus Nitinol sind, dann kann auch eine integrale Verbindung der proximalen Enden jedes ersten Drahtabschnitts mit dem inneren Schlauch in Frage kommen, indem der zumindest eine erste Drahtabschnitt aus zumindest einem Teil der Metallfaserenden der Faserarmierung des inneren Schlauchs gebildet wird.

Ein erfindungsgemäßes Verfahren zur Herstellung eines erfindungsgemäßen medizinischen Instruments sieht die folgenden Schritte vor:
- Bilden eines Werkzeugs aus zumindest zwei Drahtabschnitten zu einem Drahtgebilde und Bereitstellen einer Katheteranordnung aus einem äußeren Schlauchs und einem koaxial dazu angeordneten und relativ dazu beweglichen inneren Schlauch,
- Befestigen eines proximalen Endes zumindest eines ersten Drahtabschnitts an dem distalen Ende des inneren Schlauchs und
- Befestigen eines proximalen Endes zumindest eines zweiten Drahtabschnitts an dem distalen Ende des äußeren Schlauchs, sodass das Werkzeug zum Öffnen und Schließen durch die Relativbewegung zwischen dem äußeren Schlauch und dem inneren Schlauch ohne Führungsdraht betätigbar ist, und
- koaxial Einführen des Lichtwellenleiters in und durch den inneren Schlauch, sodass ein Austrittsende des Lichtwellenleiters koaxial in einen von dem Werkzeug begrenzten Raum mündet. Das Einführen des Lichtwellenleiters erfolgt zum Schluss, um eine Beschädigung desselben während des Zusammenbaus des Instruments zu vermeiden. Vorzugsweise kann das Einführen des Lichtwellenleiters in den inneren Schlauch in einer axial gestreckten Position der gesamten Katheteranordnung erfolgen, um eine Beschädigung sowohl des Lichtwellenleiters als auch der Schlauchanordnung zu vermeiden

In einer Weiterbildung des Verfahrens können vor dem Einführen des Lichtwellenleiters die Schritte erfolgen:
- Bereitstellen des Lichtwellenleiters ohne Buffering oder Entfernen des Bufferings von dem Lichtwellenleiter, und/oder
- Bereitstellen eines Griffs mit einem Griffkörper und zumindest einem Betätigungselement zur Betätigung des Werkzeugs, und
- Verbinden eines proximalen Endabschnitts des äußeren Schlauchs mit dem Betätigungselement, und
- Lagern eines proximalen Endabschnitt des inneren Schlauchs, der im Griff über das proximale Ende des äußeren Schlauchs hinausragt, im Griff, sodass der äußere Schlauch zur Betätigung des Werkzeugs mittels des Betätigungselements gegenüber dem inneren Schlauch axial verschiebbar ist.

Weitere Ausführungsformen sowie einige der Vorteile, die mit diesen und weiteren Ausführungsformen verbunden sind, werden durch die nachfolgende ausführliche Beschreibung unter Bezug auf die begleitenden Figuren deutlich und besser verständlich. Gegenstände oder Teile derselben, die im Wesentlichen gleich oder ähnlich sind, können mit denselben Bezugszeichen versehen sein. Die Figuren sind lediglich eine schematische Darstellung einer Ausführungsform der Erfindung.

Dabei zeigen:
- **Fig. 1**: eine Draufsicht des medizinischen Instruments in einer erfindungsgemäßen Ausführungsform,
- **Fig. 2**: eine Schnittansicht durch das medizinische Instrument aus Fig. 1 entlang Schnittlinie B-B,
- **Fig. 3**: eine vergrößerte Frontansicht von vorne auf das Steinfangkörbchen des medizinischen Instruments aus Fig. 1,
- **Fig. 4**: eine Detailschnittansicht des Details D aus Fig. 2,
- **Fig. 5**: eine schematische Darstellung einer Ausführungsform des Griffkörpers eines erfindungsgemäßen medizinischen Instruments,
- **Fig. 6**: eine perspektivische Ansicht eines alternativ ausgeführten Steinfangkörbchens eines erfindungsgemäßen medizinischen Instruments,
- **Fig. 7**: perspektivische Ansichten a), b), c) alternativ ausgeführter Steinfangkörbchen eines erfindungsgemäßen medizinischen Instruments,
- **Fig. 8**: perspektivische Ansichten a), b) alternativ ausgeführter Werkzeuge mit einer Drahtschlinge eines erfindungsgemäßen medizinischen Instruments,
- **Fig. 9**: eine perspektivische Ansicht einer Einsatzsituation eines erfindungsgemäßen medizinischen Instruments mit der aus einem Arbeitskanal eines endoskopischen Instruments herausgeführten Arbeitsspitze mit Steinfangkörbchen und Laser.

Die Erfindung betrifft ein medizinisches Instrument, das ein Werkzeug und einen Lichtwellenleiter zugleich einsetzt. Dabei kann das Werkzeug beispielsweise ein Greifwerkzeug wie ein Steinfangkörbchen sein, das mit einem koaxial angeordneten Lichtwellenleiter, z. B. einer Laserfaser als Lithotripter, kombiniert wird.

Der Lichtwellenleiter kann aufgrund der verwendeten Thuliumlasertechnologie und der erfindungsgemäßen Ausführung ohne Buffering und aufgrund der Betätigung des Werkzeugs ohne Führungsdraht mit deutlich verringertem Durchmesser gegenüber herkömmlichen Koaxial-Lasern ausgeführt werden, und weist damit eine deutlich erhöhte Flexibilität bei zugleich guter Dauer- und Biegestabilität auf. Dabei übernimmt der innere Schlauch der Katheteranordnung eine Mehrfachfunktion und sorgt neben dem Schutz des Lichtwellenleiters für die Stabilität der Katheteranordnung und bildet gleichzeitig einen Teil des Betätigungsmechanismus' des Werkzeugs.

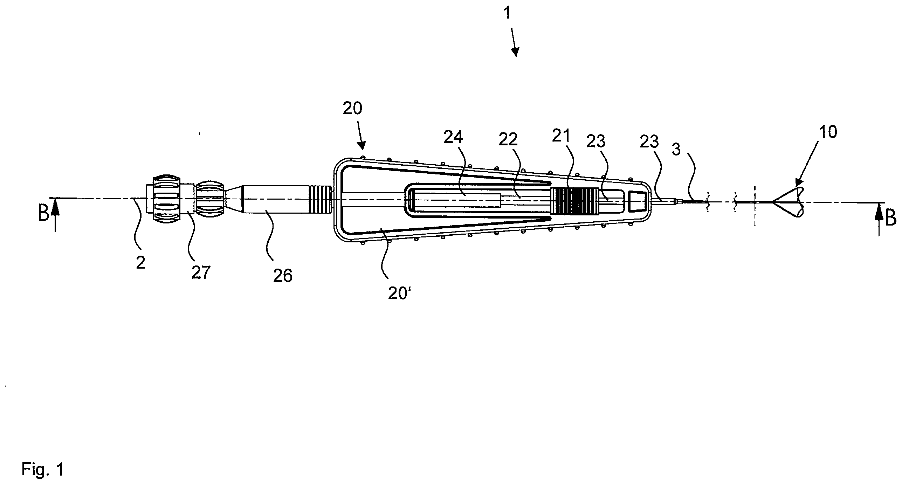

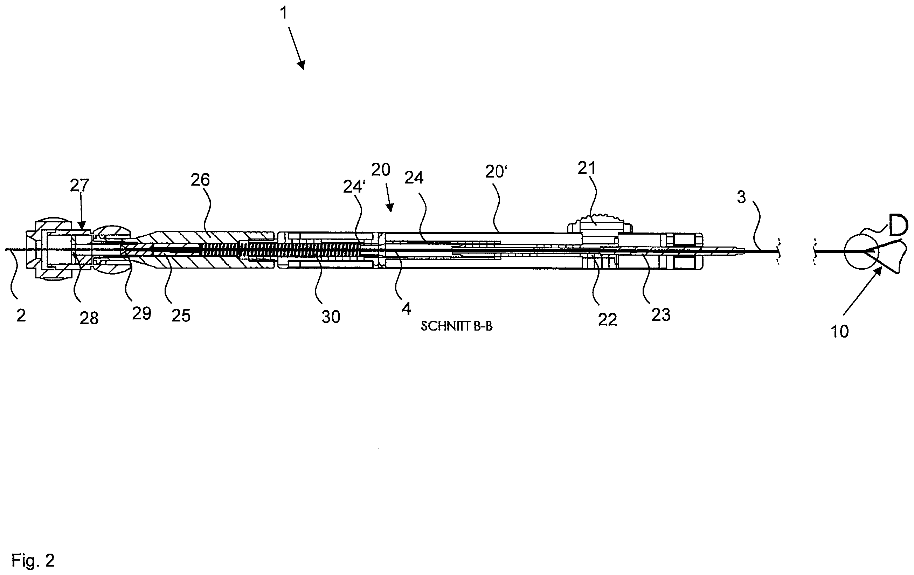

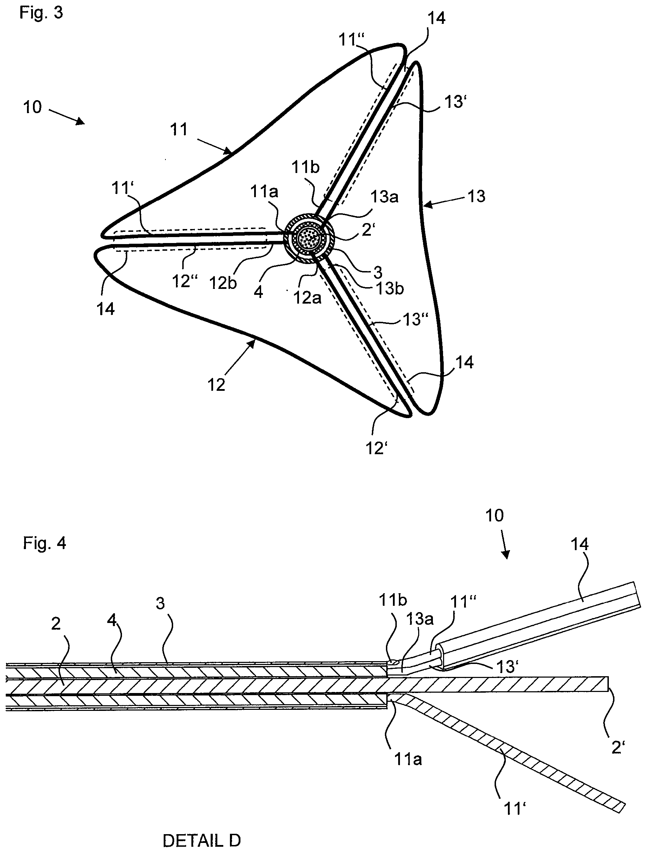

Wesentlich für den Verzicht eines Führungsdrahts bei einem medizinischen Instrument 1, wie es in **Fig. 1** und **2** gezeigt ist, ist die Befestigung des aus verbundenen Drahtabschnitten gebildeten Werkzeugs 10 mit den proximalen Drahtenden an den distalen Enden des äußeren und inneren Schlauchs 3, 4 der Katheteranordnung. **Fig. 3** und **4** zeigen dies im Detail am Beispiel eines offenen Steinfangkörbchens 10 des Instruments 1 aus **Fig. 1****,****2** mit drei Drahtschlingen 11, 12, 13. Die drei Drahtschlingen 11, 12, 13 bilden jeweils in der Draufsicht annähernd ein Dreieck mit einem ersten Drahtabschnitt 11', 12', 13', dessen proximales Ende 11a, 12a, 13a am inneren Schlauch 4 befestigt ist, und mit einem zweiten Drahtabschnitt 11", 12", 13", dessen proximales Ende 11b, 12b, 13b mit dem äußeren Schlauch 3 verbunden ist. Ein die ersten Drahtabschnitte 11', 12', 13' und zweiten Drahtabschnitte 11", 12", 13" jeweils verbindender Drahtabschnitt jeder Drahtschlinge 11, 12, 13 ist hierbei nicht mit Bezugszeichen bezeichnet.

In **Fig. 3** ist aus darstellerischen Gründen ein deutlicher Abstand zwischen dem äußeren Schlauch 3 und dem inneren Schlauch 4 zu sehen, um das Prinzip der Verbindungen der proximalen Enden 11a, 12a, 13a, 11b, 12b, 13b mit dem jeweiligen äußeren und dem inneren Schlauch 3, 4 deutlicher herauszustellen. Zwischen dem äußeren und inneren Schlauch 3, 4 ist ebenso wie zwischen dem inneren Schlauch 3 und dem Lichtwellenleiter 2 kein wesentliches radiales Spiel vorgesehen. Die Durchmesser sind so angepasst ausgewählt, dass der Lichtwellenleiter 2 in den inneren Schlauch 4 eingeführt und ggf. darin axial verschoben werden kann und dass der äußere Schlauch 3 relativ zum inneren Schlauch axial beweglich ist, aber die koaxiale Anordnung von äußerem und innerem Schlauch 3, 4 mit dem Lichtwellenleiter 2 gewährleistet ist, die die Position des Werkzeugs 10 festlegen und die koaxiale Mündung des Lichtwellenleiters in das Werkzeug 10 ermöglichen.

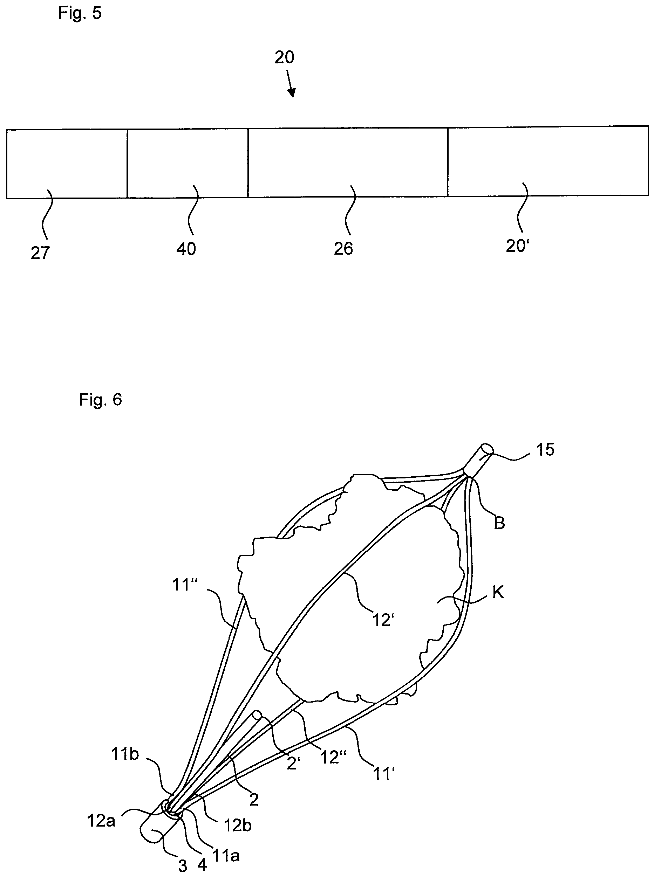

Damit das Steinfangkörbchen 10 mit der Katheterschlauchanordnung ohne Führungsdraht betätigt werden kann, sind die proximalen Enden 11a, 12a, 13a der ersten Drahtabschnitte 11', 12', 13' jeder Drahtschlinge 11, 12, 13 an dem inneren Schlauch 4 befestigt, in dem die Laserfaser 2 koaxial geführt ist, sodass das Austrittsende 2' im Mittelpunkt des Steinfangkörbchens 10 liegt und ein austretende Laserstrahl koaxial verläuft, und die proximalen Enden 11b, 12b, 13b der zweiten Drahtabschnitte 11", 12", 13" sind an dem äußeren Schlauch 3 befestigt. Damit das dreiarmige offene Steinfangkörbchen 10 in gewünschter Weise öffnet und schließt, wird jeweils ein erster Drahtabschnitt 11', 12', 13' jeder Drahtschlinge 11, 12, 13 zusammen mit dem zweiten Drahtabschnitt 12", 13", 11" der jeweils benachbarten Drahtschlinge 12, 13, 11 in einer gemeinsamen Hülle 14 geführt. Die Hülle 14, die eine höhere Lebensdauer und bei geeigneter Farb- bzw. Materialwahl eine bessere Sichtbarkeit ermöglicht, ist im gezeigten Beispiel nicht an dem äußeren Schlauch 3 befestigt. Eine solche Befestigung, z. B. mittels eines Schrumpfschlauchabschnitts, kann aber durchaus vorgesehen sein, um ein Verrutschen der Hüllen 14 zu verhindern. Für andere Werkzeuge oder geschlossene Steinfangkörbchen 10 (vgl. **Fig. 6** bis **9**) ist eine solche Führungshülle 14 nicht erforderlich.

Die hier im Detail gezeigte Anordnung der proximalen Drahtenden am inneren Schlauch und am äußeren Schlauch lässt sich auf andere Ausführungsformen des Werkzeugs mit einer abweichenden Anzahl von Drahtschlingen übertragen. Wesentlich dabei ist, dass von den beiden Enden jeder Drahtschlinge jeweils eines am inneren Schlauch und eines am äußeren Schlauch befestigt ist, um die Drahtschlingen zur Betätigung des Werkzeugs durch eine Relativverschiebung der Schlauchanordnung verformen zu können.

Generell können die Möglichkeiten zur Befestigung der proximalen Enden der Drahtabschnitte an dem äußeren und dem inneren Schlauch von dem Material der Schläuche abhängen, wobei die Drahtabschnitte vorzugsweise aus Metall, besonders bevorzugt aus Nitinol sind, sodass sie mit einer Vorspannung für die geöffnete Position des Werkzeugs ausgeführt sein können.

Der äußere Schlauch, an dem die proximalen Enden der zweiten Drahtabschnitte befestigt sind, ist aus einem Kunststoffmaterial, sodass die proximalen Enden der zweiten Drahtabschnitte in das distale Schlauchende eingeschmolzen werden können, wenn es sich bei dem Kunststoffmaterial um einen schmelzbaren Thermoplasten handelt. Da aber der äußere Schlauch vorzugsweise aus einem hitzebeständigen Kunststoff wie Polyimiden gefertigt sein kann, die z. T. nicht schmelzbar sind, können die proximalen Enden der zweiten Drahtabschnitte mittels Klebstoff und/oder eines zusätzlichen Schrumpfschlauchabschnitts (nicht dargestellt) an dem äußeren Schlauch befestigt werden.

Die proximalen Enden der ersten Drahtabschnitte können grundsätzlich auf dieselbe Weise, d. h. mittels Klebstoff und/oder eines zusätzlichen Schrumpfschlauchabschnitts, an dem inneren Schlauch befestigt werden, der - im Gegensatz zum äußeren Schlauch - eine Faserarmierung zum Biegeschutz der darin geführten Laserfaser aufweisen kann. Zur Verringerung der Reibung am äußeren Schlauch für ein besseres Abgleiten bei der Relativbewegung zwischen dem äußeren und dem inneren Schlauch kann die Faserarmierung zumindest auf der Außenseite mit einer reibungsverringernden Kunststoffbeschichtung versehen sein. Hierzu kann ein Kunststoffmaterial mit einem geringen Reibungskoeffizienten wie beispielsweise Polytetrafluorethylen oder ähnliches verwendet werden. Vorteilhaft ist dabei die Haftreibung genauso groß wie die Gleitreibung, sodass die Relativbewegung zwischen äußerem und innerem Schlauch ruckfrei stattfinden kann.

Durch die Faserarmierung bieten sich zur Verbindung der proximalen Enden der ersten Drahtabschnitte mit dem inneren Schlauch weitere Möglichkeiten an: Beispielsweise können offene Faserenden am distalen Ende des inneren Schlauchs durch Reibschluss mit den proximalen Enden der ersten Drahtabschnitte verbunden werden, d. h. durch Verschlingung, Verknotung, Verflechtung etc. Die Faserenden können auch durch Stoffschluss mit den ersten ersten Enden verbunden werden, beispielsweise durch Kleben. Sind die Fasern der Faserarmierung zumindest teilweise aus Metall, kommt auch Verschweißen oder Löten zur Verbindung der Faserenden mit den proximalen Enden der ersten Drahtabschnitte in Frage. Handelt es sich bei dem Metall der Armierungsfasern um dasselbe Metall, wie es für die Drahtschlingen vorgesehen ist, kann eine integrale Verbindung zwischen der Faserarmierung und den proximalen Enden der ersten Drahtabschnitte vorliegen, indem die ersten Drahtabschnitte aus den Metallfaserenden der Fasertextilarmierung des inneren Schlauchs oder zumindest aus einem Teil dieser Metallfaserenden gebildet werden.

**Fig. 1** und **2** verdeutlichen die Betätigung des Steinfangkörbchens 10 am Griff 20, der dazu einen gegen den Griffkörper 20' axial verschiebbaren Griffschieber 21 aufweist. Der Griff 20 umfasst ferner einen Griffkörperaufsatz 26 und einen axialen Endabschnitt (Tuohy-Borst-Adatper) 27, die sich jeweils am proximalen Ende des Griffkörpers 20' bzw. des Aufsatzes 26 anschließen und zur Lagerung des inneren Schlauchs 4 in einer Fixierhülse 25 und zur Abdichtung der Laserfaser 2 mit einer Dichtlippe 28 dienen. Die Laserfaser 2 erstreckt sich koaxial durch die gesamte Katheteranordnung und den Griff 20 und aus dem proximalen axialen Endabschnitt 27, um mit einer Laserlichtquelle (Thuliumlaser - nicht dargestellt) verbunden werden zu können.

Wie aus der Schnittdarstellung in **Fig. 2** zu ersehen ist, erfolgt die Befestigung des äußeren Schlauchs 3 an dem Griffschieber 21 mittels einer Verbindungshülse 23, die einen proximalen Endabschnitt des äußeren Schlauchs 3 umgibt und daran befestigt, z. B. geklebt ist. Die Verbindungshülse 23 ist in einer Gleithülse 22 gelagert, die mit dem Griffschieber 21 verbunden ist. In diesem Beispiel wird die Gleithülse 22 zudem in einer Führungshülse 24 geführt, die proximal zur Gleithülse 22 im Griffkörper 20' angeordnet ist. Durch diese Führungshülse 24 erstreckt sich der innere Schlauch 4 bis zu der Fixierhülse 25, in der ein proximaler Endabschnitt des inneren Schlauchs 4 befestigt ist. Die Fixierhülse 25 wird in einem Griffkörperaufsatz 26 gelagert, der sich axial am proximalen Ende des Griffkörpers 20' anschließt. Griffkörper 20' und Griffkörperaufsatz 26 können dafür korrespondierende Gewindeabschnitte aufweisen. Die Fixierhülse 25 ist gegenüber dem verschiebbaren äußeren Schlauch 4 ortsfest, aber mit einem Überlastschutz gelagert. Dies wird durch eine Schraubenfeder 30 bereitgestellt, an deren proximalen Ende die Fixierhülse 25 anliegt und deren distales Ende sich an dem Ringabsatz 24' abstützt, der durch das proximale Ende der Führungshülse 24 gebildet wird. Die Eigenspannung bzw. eine Vorspannung der Feder 30 bestimmt die Kraft, bis zu welcher die Fixierhülse 25 ortsfest gelagert bleibt. Um eine Beschädigung des Steinfangkörbchens 10 bei zu großen Zugkräften zu vermeiden, z. B. wenn ein sehr großes eingefangenes Konkrement ein weiteres Schließen des Körbchens 10 bei Vorschieben des Griffschiebers 21 verhindert, bewirken die dabei auf den inneren Schlauch 4 wirkenden Zugkräfte über die Fixierhülse 25 eine Komprimierung der Feder 30 - d. h. der innere Schlauch 4 folgt zur Entlastung der Drahtschlingen der Bewegung des äußeren Schlauchs 3.

Dabei liegt das Steinfangkörbchen 10 in der Darstellung in geöffneter Stellung vor. Durch Verschieben des Griffschiebers 21 in distale Richtung, d. h. nach vorne in Richtung des Steinfangkörbchens 10, verformt sich das Steinfangkörbchen 10 in die geschlossene Stellung durch die Verschiebung des äußeren Schlauchs 3 gegenüber dem inneren Schlauch 4 mit den jeweils daran befestigten Drahtschlingenenden.

In anderen Ausführungsformen sind aber auch davon abweichende Betätigungsvarianten denkbar.

Im Griffkörperaufsatz 26 ist proximal zur Fixierhülse 25, in der das proximale Ende des inneren Schlauchs 4 befestigt ist, eine Einführhilfe 29 angeordnet, um das Einführen der Laserfaser 2 in den inneren Schlauch 4 beim Zusammenbau zu erleichtern. Die Einführhilfe 29 weist dazu an ihrem proximalen Ende eine sich - auf den Innendurchmesser des inneren Schlauchs 4 - verjüngende trichterartige Öffnung auf. Zur Fixierung und Abdichtung der Laserfaser 2 ist am proximalen Ende des Griffkörperaufsatzes 26 ein axialer Endabschnitt 27 wie ein Tuohy-Borst-Adapter angeordnet, und beispielsweise mittels korrespondierender Gewindeabschnitte befestigt. Für die Fixierung und Abdichtung der Laserfaser 2 sorgt dabei eine radiale Dichtlippe 28, deren Öffnungsdurchmesser sich durch Drehung eines Gehäuseabschnitts des Endabschnitts 27 verstellen lässt.

Der in **Fig. 1** und **2** dargestellte Griff ist lediglich beispielhaft. Abweichungen im Design und Modifikationen von Funktionsdetails sind innerhalb des Schutzumfangs möglich. So sind Alternativen zum Griffschieber als Betätigungselement zum Verschieben des äußeren Schlauchs wie z. B. ein Stellrad, das durch Übertragungsmittel die Rotationsbewegung in axiale Translation übersetzt, ebenso denkbar wie Varianten, bei denen der innere Schlauch gegenüber dem äußeren Schlauch verschoben wird, sodass die Elemente zur Betätigung und zur ortsfesten, aber überlastgesicherten Lagerung entsprechend umgekehrt ausgeführt sein müssen.

**Fig. 5** zeigt in schematischer Weise eine Ausführung eines Griffs 20, der eine Verschiebung der Laserfaser 2 unabhängig von der Betätigung eines Werkzeugs 10 wie das Öffnen und Schließen eines Steinfangkörbchens gestattet, sodass die Laserfaser 2 separat manipuliert werden kann. Dazu weist der Griff 20 ein zweites Betätigungselement 40 auf, das im dargestellten Beispiel zwischen dem Griffkörperaufsatz 26, in dem die Einführhilfe 29 und die Fixierhülse 25 vorliegen, und dem axialen Endabschnitt 27 mit der Dichtlippe 28 vorgesehen ist. Hierzu kann ein weiterer Griffabschnitt zur Integration des Betätigungselements 40 eingefügt sein, das eine Verschiebevorrichtung umfasst oder damit verbunden ist, um die Laserfaser 2 axial zu verschieben. Hierzu kommen Strukturen wie Griffschieber oder Stellrad mit Übertragungsmitteln in Frage ähnlich denen, die zur Verschiebung des äußeren Schlauchs 4 vorgesehen sind.

**Fig. 6** bis **9** zeigen alternative Werkzeuge 10 eines medizinischen Instruments 1, während in **Fig. 1** bis **4** ein offenes Steinfangkörbchen 10 mit einer dreiarmigen Ausführung als Werkzeug 10 des medizinischen Instruments 1 gezeigt ist. Dieses offene, dreiarmige Körbchen, das eine besonders bevorzugte Ausführungsform darstellt, kombiniert die Vorteile eines herkömmlichen Körbchens wie das sichere Fangen von Steinen und Fragmenten mit denen eines Greifers, der eine einfache Neupositionierung von Steinen, gestattet, und stellt damit ein optimales Werkzeug für die Steinentfernung dar. Diesem Steinfangkörbchen 10 wie auch den anderen Werkzeugen 10 der **Fig. 6** bis **9** kann durch ein Flachdrahtdesign der Drahtschlingen eine hohe Dauerstabilität verliehen werden, da das Flachdrahtdesign eine höhere Radialkraft bewirkt, die für eine verbesserte Funktionalität sorgt.

In **Fig. 6** ist als Werkzeug 10 ein geschlossenes Steinfangkörbchen 10 mit einem gefangenen Stein (Konkrement) K gezeigt. Dadurch, dass der Lichtwellenleiter 2 und die Katheteranordnung aus innerem Schlauch 4 und äußerem Schlauch 3 koaxial angeordnet sind, mündet der Lichtwellenleiter 2 exakt mittig bzw. koaxial in dem Greifwerkzeug 10, was die Wahrscheinlichkeit, eine Werkzeugstruktur zu treffen, wenn der mit dem Werkzeug 10 gegriffene Gegenstand K mit einem aus dem Lichtwellenleiter 2 austretenden Laserstrahl bestrahlt wird, deutlich verringert. Das Steinfangkörbchen 10 in **Fig. 6** hat eine gerade Form, die dem Steinfangkörbchen 10 eine größtmögliche Aufstellkraft verschafft, mit einer kappenartigen Spitze 15, in der die Drahtabschnitte 11', 11", 12', 12" zur Bildung des Drahtgebildes aus quasi zwei symmetrischen Drahtschlingen in einem Bereich B gegenüber dem Austrittsende 2' des Lichtwellenleiters 2 verbunden sind. Dabei sind die ersten Drahtelemente 11', 12' an den proximalen Enden 11a, 12a mit dem inneren Schlauch 4 verbunden, und die zweiten Drahtelemente 11", 12" sind an den Enden 11b, 12b mit dem äußeren Schlauch 3 verbunden.

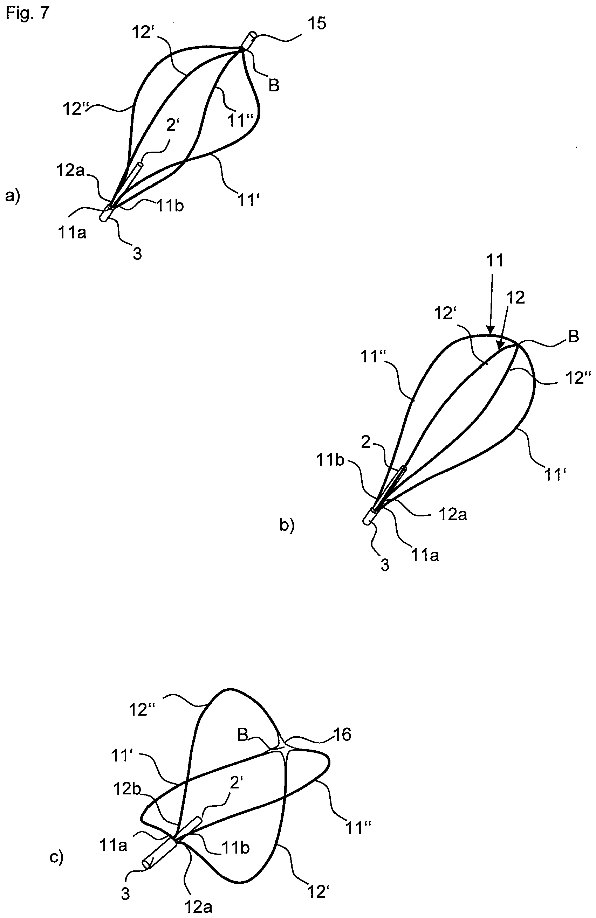

**Fig. 7** zeigt drei weitere Ausführungsformen a), b), c) eines geschlossenen Steinfangkörbchens 10, wobei hier die Details der Befestigung der Drahtschlingenenden an dem äußeren Schlauch 3 und dem inneren Schlauch (nicht dargestellt) der Übersichtlichkeit wegen nicht dargestellt sind. Das Steinfangkörbchen 10 in **Fig. 7a**) entspricht dem Körbchen aus **Fig. 6****,** außer dass es eine helikale Form aufweist, d. h. dass die Drahtabschnitte 11', 11", 12', 12" sich nicht gerade zur Spitze 15 erstrecken, sondern einen gewunden Verlauf haben. Die helikale Form ermöglicht ein leichteres Fangen eines Steins.

Alternativ zu einer Ausführung aus vier separaten Drahtabschnitten 11', 11", 12', 12" können die Drahtschlingen eines Steinfankörbchens 10 mit Spitze 15 aus einem einzigen Draht gefertigt sein. Dabei werden die Schlingen durch drei gleichmäßig beabstandete spitzwinklige Biegestellen in alternierenden Richtungen des einzigen Drahts gebildet, die diesen somit in vier zusammenhängende Drahtabschnitte 11', 11", 12', 12" unterteilen, von denen jeweils zwei benachbarte Drahtabschnitte 11', 11", 12', 12" jeweils eine der Drahtschlingen bilden. Bei der Anordnung eines solchen geschlossenen Steinfangkörbchens 10 aus einem einzigen Draht an dem distalen Ende der Katheteranordnung bestehen zwei Möglichkeiten. Entweder werden die erste und dritte Biegestelle in dem Bereich B, der dem Austrittsende 2' des Lichtwellenleiters 2 gegenüberliegt, angeordnet und miteinander in der Spitze 15 verbunden, sodass die freien Drahtenden des einzigen Drahtes proximale Enden 11a, 12a erster Drahtabschnitte 11', 12' oder proximale Enden 11b, 12b zweiter Drahtabschnitte 11", 12" bilden, die an einem von äußerem Schlauch 3 und innerem Schlauch befestigt sind, wobei die zweite Biegestelle die jeweils anderen proximalen Enden 11b, 12b zweiter Drahtabschnitte 11", 12" oder die proximalen Enden 11a, 12a erster Drahtabschnitte 11', 12' bildet, die an dem anderen von äußerem Schlauch 3 und innerem Schlauch befestigt sind. Oder alternativ können die erste Biegestelle die proximalen Enden 11a, 12a der ersten Drahtabschnitte 11', 12' und die dritte Biegestelle die proximalen Enden 11b, 12b der zweiten Drahtabschnitte 11", 12" (oder umgekehrt die erste Biegestelle die proximalen Enden 11b, 12b der zweiten Drahtabschnitte 11", 12" und die zweite Biegestelle die proximalen Enden 11a, 12a der ersten Drahtabschnitte 11',12') bilden und jeweils an dem inneren Schlauch und dem äußeren Schlauch 3 befestigt sein, während die freien Enden des einzigen Drahts und die zweite Biegestelle in dem Bereich B, der dem Austrittsende 2' des Lichtwellenleiters 2 gegenüberliegt, in der Spitze 15 verbunden sind.

**Fig. 7b)** und **7c****)** zeigen jeweils ein spitzenloses Steinfangkörbchen 10, das mit dem abgerundeten Verbindungsbereich B eine gewebeschonende Führung ermöglicht. In den dargestellten Beispielen ist das Steinfangkörbchen 10 aus zwei Drahtschlingen 11, 12 mit den jeweils einstückig verbundenen Drahtabschnitten 11', 11" und 12', 12" gebildet, wobei die proximalen Enden 11a, 12a der ersten Drahtabschnitte 11', 12' mit dem inneren Schlauch (nicht dargestellt) und die proximalen Enden 11b, 12b der zweiten Drahtabschnitte 11", 12" mit dem äußeren Schlauch 3 verbunden sind. Das Steinfangkörbchen 10 in **Fig. 7c****)** weist dabei im Verbindungsbereich B eine Verbindungsstelle bzw. -fläche 16 in Form einer Raute mit konkav gerundeten Seiten auf. Diese Verbindungsstelle 16 kann in geeigneter Färbung für eine bessere endoskopische Sicht sorgen.

Abweichend von den dargestellten Beispielen können Körbchen mit Spitze auch aus zwei Drahtschlingen oder mehr gebildet werden und Körbchen ohne Spitze können aus vier oder mehr separaten Drahtabschnitten oder einem einzigen Draht gebildet werden. Weiter abweichend von den dargestellten Beispielen können geschlossene Steinfangkörbchen auch eine unsymmetrische Anzahl von ersten und zweiten Drahtabschnitten aufweisen, deren proximale Enden dann entsprechend unsymmetrisch an dem äußeren und inneren Schlauch angeordnet sind. So kann ein Steinfangkörbchen aus vier Drahtabschnitten mit einem ersten Drahtabschnitt am inneren Schlauch und mit den drei anderen, zweiten Drahtabschnitten am äußeren Schlauch befestigt sein, oder umgekehrt. Ferner können geschlossene Steinfangkörbchen auch aus einer ungeraden Anzahl von Drahtabschnitten gebildet werden. Bei einem Steinfangkörbchen aus drei Drahtabschnitten z. B. kann ein erster Drahtabschnitt am proximalen Ende mit dem inneren Schlauch verbunden sein und die zwei anderen, zweiten Drahtabschnitte mit dem äußeren Schlauch, oder umgekehrt, zwei erste Drahtabschnitte mit dem inneren Schlauch und ein zweiter Drahtabschnitt mit dem äußeren Schlauch. Bei steigender Anzahl der Drahtabschnitte wächst die Anzahl der möglichen Anordnungsvarianten entsprechend, die daher hier nicht weiter ausgeführt werden sollen, aber sich für den Fachmann ohne weiteres ergeben.

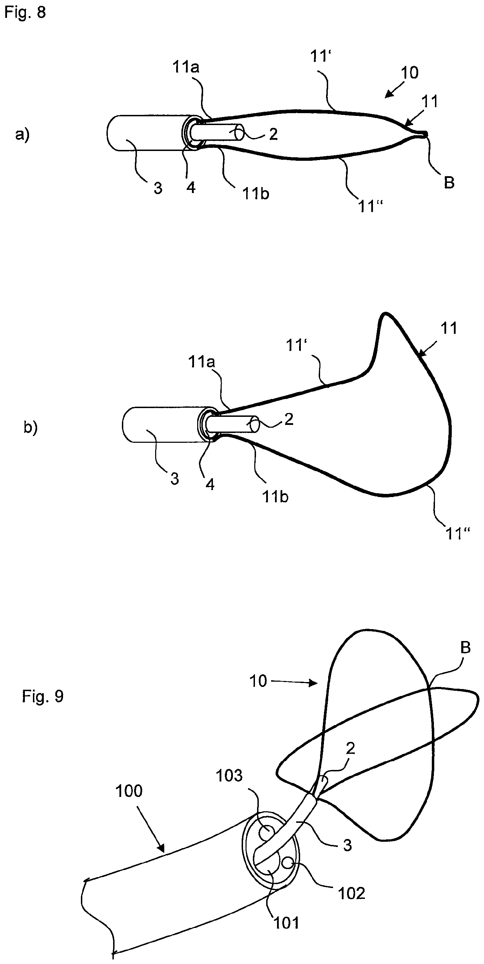

**Fig. 8** zeigt zwei Beispiele a) und b) mit alternativen Werkzeugen 10 als Arbeitsspitze eines erfindungsgemäßen medizinischen Instruments. Dabei besteht das Werkzeug 10 aus einer Drahtschlinge 11, deren erster Drahtabschnitt 11' am proximalen Ende 11a an dem inneren Schlauch 4 und deren zweiter Drahtabschnitt 11" am proximalen Ende 11b an dem äußeren Schlauch 3 der Katheteranordnung befestigt ist. Auch hier mündet die Laserfaser 2 mittig in den von der Drahtschlinge 11 begrenzten Raum, der in **Fig. 8a****)** im Wesentlichen als flächiges Oval ausgebildet ist. Nicht dargestellt sind polygonale Schlingenformen, wie z. B. hexagonal geformte Schlingen. **Fig. 8b****)** zeigt eine sichelförmige Schlinge 11, die einen schalenartigen Raum begrenzt, in den die Laserfaser 2 koaxial mündet. Die Drahtschlinge 11 kann aus einem einstückigen Draht mit den zwei zusammenhängenden Drahtabschnitten 11'; 11" oder aus zwei miteinander verbundenen Drahtabschnitten 11'; 11" gebildet sein.

In **Fig. 9** ist eine Einsatzsituation eines erfindungsgemäßes medizinischen Instruments mit Steinfangkörbchen 10 dargestellt, das durch einen Arbeitskanal 101 eines Einführschlauchs 100 eines Ureteroskops zum Beispiel an einer Einsatzstelle geöffnet wurde. Im gezeigten Beispiel weist der Einführschlauch 100 zudem eine Beleuchtungsvorrichtung 103 und einen Kamerasensor 102 auf, durch die der Anwender die Einsatzstelle beobachten kann. Durch die aufgrund der verwendeten Thuliumlasertechnologie kleinere Dimension der Katheteranordnung mit dem koaxial angeordneten Lichtwellenleiter 2, der mit einem Außendurchmesser (ohne Buffering) von 150 µm bis 200 µm (d. h. 130 µm bis 180 µm Kerndurchmesser und 10 µm Cladding) ausgebildet sein kann, bleibt dem Anwender mehr Handlungsspielraum im Arbeitskanal 101 und es kann ein maximaler Spülfluss beim Spülen erzielt werden. Zusätzlich ermöglicht die Thuliumlasertechnologie eine effizientere Lithotripsie bei höheren Pulsgeschwindigkeiten. In Bezug auf die zum Zertrümmern benötigte Laserleistung könnten theoretisch Laserfasern mit noch kleinerem Durchmesser eingesetzt werden, dann allerdings leidet die Stabilität und Haltbarkeit des Instruments. Und da bereits mit Laserfaserquerschnitten von 150 µm bis 200 µm eine sehr gute Flexibilität des Instruments und ein ausreichender Spülfluss erreicht werden, stellt eine weitere Verkleinerung des Faserquerschnitts des Lichtwellenleiters zur geringfügigen weiteren Erhöhung der Flexibilität keine wesentliche Gesamtverbesserung dar, da hiermit eine deutlich eingeschränkte Stabilität und Dauerhaltbarkeit verbunden wäre.

Grundsätzlich minimiert die kombinierte Verwendung eines Steinfangkörbchens mit einem Laserlithotripter die Steinretropulsion, da der im Steinfangkörbchen gefangene Stein beim Auftreffen des Laserimpulses nicht ausweichen kann. Vorteilhaft werden aber nun durch die nun auch in einem miniaturisierten Instrument ermöglichte koaxiale Anordnung laserinduzierte Schäden an den Drahtschlingen reduziert, da der Laserimpuls immer mittig auf ein eingefangenes Konkrement auftrifft.

Zur Herstellung eines erfindungsgemäßen medizinischen Instruments werden zunächst die Katheteranordnung aus dem äußeren und dem koaxial dazu angeordneten und relativ dazu beweglichen inneren Schlauch sowie das Werkzeug aus einem mit zumindest zwei Drahtabschnitten gebildeten Drahtgebilde bereitgestellt. Zumindest ein proximales Ende der Drahtabschnitte des Drahtgebildes wird an dem distalen Ende des inneren Schlauchs und zumindest ein proximales Ende der Drahtabschnitte des Drahtgebildes wird an dem distalen Ende des äußeren Schlauchs befestigt, sodass eine Relativbewegung zwischen äußerem und innerem Schlauch ein Öffnen und Schließen des Werkzeugs bewirkt.

Die proximalen Enden des äußeren und inneren Schlauchs werden geeignet in einem Griff mit einem Betätigungselement befestigt, um die Relativbewegung zwischen dem äußeren und dem inneren Schlauch zum Öffnen und Schließen des Werkzeugs auslösen zu können. Dazu ist es sinnvoll, dass im Griff ein proximaler Endabschnitt des inneren Schlauchs über ein proximales Ende des äußeren Schlauchs hinausragt. Dabei kann beispielsweise der proximale Endabschnitt des äußeren Schlauchs mit dem Betätigungselement verbunden und der proximaler Endabschnitt des inneren Schlauchs ortsfest im Griff gelagert werden, sodass der äußere Schlauch zur Betätigung des Werkzeugs mittels des Betätigungselements gegenüber dem inneren Schlauch axial verschoben werden kann. Die ortsfeste Lagerung des proximalen Endabschnitts des inneren Schlauchs kann ggf. zum Überlastschutz als gefederte Lagerung ausgebildet sein.

Erst zum Schluss wird der Lichtwellenleiter, der vorzugsweise ohne Buffering eingesetzt wird, sodass ggf. zunächst das Buffering entfernt wird, koaxial in und durch den inneren Schlauch von dessen proximalen Ende her eingeführt, bis das Austrittsende des Lichtwellenleiters das distale Ende des inneren Schlauchs zumindest abschließt und damit koaxial in den von dem Werkzeug begrenzten Raum münden kann. Da das proximale Ende des inneren Schlauchs im Griff gelagert ist, weist dieser am proximalen Ende eine abdichtbare Öffnung zum Einführen des Lichtwellenleiters auf. Zusätzlich kann dort eine Einführhilfe mit einer sich verjüngenden Einführöffnung vorgesehen sein, um das Einfädeln in den inneren Schlauch zu erleichtern. Dabei ist zum Einführen des Lichtwellenleiters zur Vermeidung von Beschädigungen sowohl des Lichtwellenleiters selbst als auch des inneren und ggf. äußeren Schlauchs vorteilhaft, den Griff mit der Katheteranordnung axial gestreckt zu positionieren, um das Durchführen des Lichtwellenleiters zu erleichtern.

### BEZUGSZEICHENLISTE

- 1: medizinisches Instrument
- 2, 2': Lichtwellenleiter, Austrittsende
- 3: äußerer Schlauch
- 4: innerer Schlauch
- 10: Werkzeug, Steinfangkörbchen
- 11, 12, 13: Drahtschlinge
- 11a, 12a, 13a: proximales Ende des ersten Drahtabschnitts
- 11b, 12b, 13b: proximales Ende des zweiten Drahtabschnitts
- 11', 12', 13': erste Drahtabschnitte
- 11", 12", 13": zweite Drahtabschnitte
- 14: Umhüllung
- 15: Spitze
- 16: Verbindungsstelle
- 20, 20': Griff, Griffkörper
- 21: Betätigungselement / Griffschieber
- 22: Gleithülse
- 23: Verbindungshülse
- 24: Führungshülse
- 24': Ringabsatz
- 25: Fixierhülse
- 26: Griffkörperaufsatz
- 27: proximaler axialer Endabschnitt / Tuohy-Borst-Adapter
- 28: Dichtlippe
- 29: Einführhilfe für Laserfaser
- 30: Lagervorrichtung / Feder
- 40: zweites Betätigungselement
- 100: Einsatzschlauch
- 101: Arbeitskanal
- 102: Kamerasensor
- 103: Beleuchtung
- B: dem Austrittsende gegenüberliegender Bereich
- K: Konkrement

## Patentansprüche

1. Medizinisches Instrument (1) mit einem Werkzeug (10), einer Katheteranordnung und einem Griff (20), mit dem das Werkzeug (10) betätigbar ist, das ein Drahtgebilde mit zumindest zwei Drahtabschnitten (11', 11", 12', 12", 13', 13") ist und das an einem distalen Ende der Katheteranordnung angeordnet ist, die aus einem äußeren Schlauch (3) und einem koaxial dazu angeordneten inneren Schlauch (4) gebildet wird, wobei der äußere Schlauch (3) und der innere Schlauch (4) relativ zueinander beweglich sind, und wobei das medizinische Instrument (1) ferner einen Lichtwellenleiter (2) aufweist, der sich durch den inneren Schlauch (4) erstreckt und dessen Austrittsende (2') koaxial in einen von dem Werkzeug (10) begrenzten Raum mündet,
und wobei das medizinische Instrument (1) keinen Führungsdraht zur Betätigung des Werkzeugs (10) aufweist,
**dadurch gekennzeichnet, dass**
zumindest ein erster Drahtabschnitt (11', 12', 13') mit einem proximalen Ende (11a, 12a, 13a) an dem distalen Ende des inneren Schlauchs (4) und zumindest ein zweiter Drahtabschnitt (11", 12", 13") mit einem proximalen Ende (11b, 12b, 13b) an dem distalen Ende des äußeren Schlauchs (3) befestigt ist,
und
dass das Werkzeug (10) zum Öffnen und Schließen durch die Relativbewegung zwischen dem äußeren Schlauch (3) und dem inneren Schlauch (4) betätigbar ist.

2. Medizinisches Instrument (1) nach Anspruch 1,
**dadurch gekennzeichnet, dass**
der Lichtwellenleiter (2) eine Laserfaser (2) ist, die zur Leitung von Laserstrahlung eines Thuliumlasers ausgebildet ist, wobei die Laserfaser (2) kein Buffering aufweist.

3. Medizinisches Instrument (1) nach Anspruch 1 oder 2,
**dadurch gekennzeichnet, dass**
der Griff (20) einen Griffkörper (20') und zumindest ein Betätigungselement (21) zur Betätigung des Werkzeugs (10) aufweist, wobei
- ein proximaler Endabschnitt des äußeren Schlauchs (3) mit dem Betätigungselement (21) verbunden ist, und ein proximaler Endabschnitt des inneren Schlauchs (4), der im Griff (20) über das proximale Ende des äußeren Schlauchs (3) hinausragt, im Griff (20) gelagert ist, sodass der äußere Schlauch (3) zur Betätigung des Werkzeugs (10) mittels des Betätigungselements (21) gegenüber dem inneren Schlauch (4) axial verschiebbar ist, oder
- ein proximaler Endabschnitt des äußeren Schlauchs (3) im Griff (20) gelagert ist, und ein proximaler Endabschnitt des inneren Schlauchs (4), der im Griff (20) über das proximale Ende des äußeren Schlauchs (3) hinausragt, mit dem Betätigungselement (21) verbunden ist, sodass der innere Schlauch (4) zur Betätigung des Werkzeugs (10) mittels des Betätigungselements (21) gegenüber dem äußeren Schlauch (3) axial verschiebbar ist.

4. Medizinisches Instrument (1) nach Anspruch 3,
**dadurch gekennzeichnet, dass**
das Betätigungselement (21) ein Griffschieber (21) ist, der zur Bewegung des äußeren Schlauchs (3) in axialer Richtung gegen den Griffkörper (20') verschiebbar ist, und/oder
die Verbindung des Betätigungselements (21) mit dem proximalen Endabschnitt des äußeren Schlauchs (3) über eine Gleithülse (22) bereitgestellt wird, die mit dem Betätigungselement (21) verbunden ist und in der eine Verbindungshülse (23) gelagert ist, in der der äußere Schlauch (3) befestigt ist.

5. Medizinisches Instrument (1) nach Anspruch 3 oder 4,
**dadurch gekennzeichnet, dass**
die Lagerung des proximalen Endabschnitts des inneren Schlauchs (4) im Griff (20) durch eine Fixierhülse (25) bereitgestellt wird, in der der innere Schlauch (4) befestigt ist und die mit ihrem distalen Ende an einer Lagervorrichtung anliegt, die zwischen dem proximalen Ende des äußeren Schlauchs (3) und der Fixierhülse (25) in dem Griffkörper (20') oder in einem Griffkörperaufsatz (26) vorgesehen ist, der sich an ein proximales Ende des Griffkörpers (20') anschließt.

6. Medizinisches Instrument (1) nach Anspruch 5,
**dadurch gekennzeichnet, dass**
die Lagervorrichtung eine Feder (30) ist, an deren proximalen Ende die Fixierhülse (25) anliegt, wobei sich das distale Ende der Feder (30) an einem Ringabsatz (24') abstützt, der im Griffkörper (20') vorliegt.

7. Medizinisches Instrument (1) nach Anspruch 6,
**dadurch gekennzeichnet, dass**
der Ringabsatz (24') durch ein proximales Ende einer Führungshülse (24) bereitgestellt wird, die im Griffkörper (20') zur Führung der Gleithülse (22) angeordnet ist.

8. Medizinisches Instrument (1) nach zumindest einem der Ansprüche 3 bis 7,
**dadurch gekennzeichnet, dass**
der Lichtwellenleiter (2) über das proximale Ende des inneren Schlauchs (4) hinausragt und sich durch einen proximalen axialen Endabschnitt (27) des Griffs (20) aus dem Griff (20) erstreckt, wobei der proximale axiale Endabschnitt (27) eine ringförmige Dichtlippe (28) aufweist, die abdichtend um den Lichtwellenleiter (2) anliegt, wobei bevorzugt die ringförmige Dichtlippe (28) radial verstellbar ist bzw. einen radial verstellbaren Öffnungsquerschnitt aufweist.

9. Medizinisches Instrument (1) nach zumindest einem der Ansprüche 1 bis 8,
**dadurch gekennzeichnet, dass**
der Lichtwellenleiter (2) axial verschiebbar in dem inneren Schlauch (4) angeordnet ist, wobei bevorzugt der Griff (20) ein zweites Betätigungselement (40) aufweist, das eine Verschiebevorrichtung umfasst oder damit verbunden ist, die mit dem Lichtwellenleiter (2) in Wirkverbindung steht und die eine axiale Verschiebung des Lichtwellenleiters (2) bereitstellt,
und/oder
der Griff (20) eine Einführhilfe (29) mit einer sich auf einen Innenquerschnitt des inneren Schlauchs (4) verjüngenden Einführöffnung für den Lichtwellenleiter (2) in den inneren Schlauch (4) aufweist.

10. Medizinisches Instrument nach zumindest einem der Ansprüche 1 bis 9,
**dadurch gekennzeichnet, dass**
das Drahtgebilde des Werkzeugs (10)
- eine Drahtschlinge (11) ist, die aus einem ersten Drahtabschnitt (11') und einem zweiten Drahtabschnitt (11") gebildet ist, oder
- ein Steinfangkörbchen (10) aus zwei oder drei Drahtschlingen (11, 12, 13) ist, die jeweils aus einem ersten Drahtabschnitt (11', 12', 13') und einem zweiten Drahtabschnitt (11", 12", 13") gebildet sind.

11. Medizinisches Instrument nach zumindest einem der Ansprüche 1 bis 10,
**dadurch gekennzeichnet, dass**
das Drahtgebilde
- aus Nitinol ist und eine Vorspannung für die geöffnete Position des Werkzeugs (10) aufweist, und/oder
- zumindest teilweise durch Flachdraht bereitgestellt wird.

12. Medizinisches Instrument nach zumindest einem der Ansprüche 1 bis 11,
**dadurch gekennzeichnet, dass**
- der äußere Schlauch (3) aus einem hitzebeständigen Kunststoff gefertigt ist, und/oder
- der innere Schlauch (4) eine Faserarmierung mit einer reibungsverringernden Kunststoffbeschichtung aufweist, wobei die Faserarmierung vorzugsweise eine Metallfaserarmierung ist.

13. Medizinisches Instrument nach Anspruch 12,
**dadurch gekennzeichnet, dass**
- die Befestigung des proximalen Endes (11b, 12b, 13b) des zumindest einen zweiten Drahtabschnitts (11", 12", 13") an dem äußeren Schlauch (3) durch Kleben, Einschmelzen und/oder einen zusätzlichen Schrumpfschlauchabschnitt, der sich über einen Verbindungsabschnitt am Ende des äußeren Schlauchs (3) und das proximale Ende (11b, 12b, 13b) des zumindest einen zweiten Drahtabschnitts (11", 12", 13") erstreckt, bereitgestellt wird,
und/oder
- die Befestigung des proximalen Endes (11a, 12a, 13a) des zumindest einen ersten Drahtabschnitts (11', 12', 13') an dem inneren Schlauch (4)
a) durch Kleben, Einschmelzen und/oder einen zusätzlichen Schrumpfschlauchabschnitt, der sich über einen Verbindungsabschnitt am Ende des inneren Schlauchs (4) und das proximale Ende (11a, 12a, 13a) des zumindest einen ersten Drahtabschnitts (11', 12', 13') erstreckt, bereitgestellt wird, oder
b) durch eine reibschlüssige und/oder stoffschlüssige Verbindung des proximalen Endes (11a, 12a, 13a) des zumindest einen ersten Drahtabschnitts (11', 12', 13') mit Faserenden der Faserarmierung bereitgestellt wird, die an dem distalen Ende des inneren Schlauchs (4) vorliegen, oder
c) durch eine integrale Verbindung der zumindest einen ersten Drahtabschnitts (11', 12', 13') mit den Metallfasern der Fasertextilarmierung des inneren Schlauchs (4) bereitgestellt wird, wobei der zumindest eine erste Drahtabschnitt (11', 12', 13') aus zumindest einem Teil der Metallfaserenden der Fasertextilarmierung des inneren Schlauchs (4) gebildet wird.

14. Verfahren zur Herstellung eines medizinischen Instruments (1) nach zumindest einem der Ansprüche 1 bis 13,
**umfassend die Schritte**
- Bilden eines Werkzeugs (10) aus zumindest zwei Drahtabschnitten (11', 11", 12', 12", 13', 13") zu einem Drahtgebilde und Bereitstellen einer Katheteranordnung aus einem äußeren Schlauch (3) und einem koaxial dazu angeordneten und relativ dazu beweglichen inneren Schlauch (4),
- Befestigen eines proximalen Endes (11a, 12a, 13a) zumindest eines ersten Drahtabschnitts (11', 12', 13') an dem distalen Ende des inneren Schlauchs (4) und
- Befestigen eines proximalen Endes (11b, 12b, 13b) zumindest eines zweiten Drahtabschnitts (11 ", 12", 13") an dem distalen Ende des äußeren Schlauchs (3), sodass das Werkzeug (10) zum Öffnen und Schließen durch die Relativbewegung zwischen dem äußeren Schlauch (3) und dem inneren Schlauch (4) ohne Führungsdraht betätigbar ist, und
- koaxial Einführen des Lichtwellenleiters (2) in und durch den inneren Schlauch (4), sodass ein Austrittsende (2') des Lichtwellenleiters (2) koaxial in einen von dem Werkzeug (10) begrenzten Raum mündet.

15. Verfahren nach Anspruch 14,
**umfassend die Schritte**
vor dem Einführen des Lichtwellenleiters (2)
- Bereitstellen des Lichtwellenleiters (2) ohne Buffering oder Entfernen des Bufferings von dem Lichtwellenleiter (2), und/oder
- Bereitstellen eines Griffs (20) mit einem Griffkörper (20) und zumindest einem Betätigungselement (21) zur Betätigung des Werkzeugs (10), und
- Verbinden eines proximalen Endabschnitts des äußeren Schlauchs (3) mit dem Betätigungselement (21), und
- Lagern eines proximalen Endabschnitts des inneren Schlauchs (4), der im Griff (20) über das proximale Ende des äußeren Schlauchs (3) hinausragt, im Griff (20), sodass der äußere Schlauch (3) zur Betätigung des Werkzeugs (10) mittels des Betätigungselements (21) gegenüber dem inneren Schlauch (4) axial verschiebbar ist.

## Claims

1. A medical instrument (1) comprising a tool (10), a catheter assembly, and a handle (20), by means of which the tool (10) can be actuated, and which is a wire formation comprising at least two wire sections (11', 11", 12', 12", 13', 13"), and which is arranged at a distal end of the catheter assembly, which is formed from an outer tube (3) and an inner tube (4), which is arranged coaxially thereto, wherein the outer tube (3) and the inner tube (4) are movable relative to one another, and wherein the medical instrument (1) further has an optical waveguide (2), which extends through the inner tube (4) and the exit end (2') of which opens coaxially into a space, which is limited by the tool (10),
and wherein the medical instrument (1) does not have a guide wire for actuating the tool (10),
**characterised in that**
at least a first wire section (11', 12', 13') is fastened with a proximal end (11a, 12a, 13a) to the distal end of the inner tube (4), and at least a second wire section (11", 12", 13") is fastened with a proximal end (11b, 12b, 13b) to the distal end of the outer tube (3),
and
that the tool (10) can be actuated to open and close by means of the relative movement between the outer tube (3) and the inner tube (4).

2. The medical instrument (1) according to claim 1,
**characterised in that**
the optical waveguide (2) is a laser fibre (2), which is formed to guide laser radiation of a thulium laser, wherein the laser fibre (2) does not have a buffering.

3. The medical instrument (1) according to claim 1 or 2,
**characterised in that**
the handle (20) has a handle body (20') and at least one actuating element (21) for actuating the tool (10), wherein
- a proximal end section of the outer tube (3) is connected to the actuating element (21), and a proximal end section of the inner tube (4), which protrudes in the handle (20) beyond the proximal end of the outer tube (3), is supported in the handle (20), so that the outer tube (3) is axially displaceable with respect to the inner tube (4) in order to actuate the tool (10) by means of the actuating element (21), or
- a proximal end section of the outer tube (3) is supported in the handle (20), and a proximal end section of the inner tube (4), which protrudes in the handle (20) beyond the proximal end of the outer tube (3), is connected to the actuating element (21), so that the inner tube (4) is axially displaceable with respect to the outer tube (3) in order to actuate the tool (10) by means of the actuating element (21).

4. The medical instrument (1) according to claim 3,
**characterised in that**
the actuating element (21) is a handle slide (21), which is displaceable in the axial direction against the handle body (20') in order to move the outer tube (3). and/or
the connection of the actuating element (21) to the proximal end section of the outer tube (3) is provided via a sliding sleeve (22), which is connected to the actuating element (21) and in which a connecting sleeve (23) is supported, in which the outer tube (3) is fastened.

5. The medical instrument (1) according to claim 3 or 4,
**characterised in that**
the support of the proximal end section of the inner tube (4) in the handle (20) is provided by means of a fixing sleeve (25), in which the inner tube (4) is fastened, and which bears with its distal end against a support device, which is provided between the proximal end of the outer tube (3) and the fixing sleeve (25) in the handle body (20') or in a handle body attachment (26), which adjoins a proximal end of the handle body (20').

6. The medical instrument (1) according to claim 5,
**characterised in that**
the support device is a spring (30), against the proximal end of which the fixing sleeve (25) bears, wherein the distal end of the spring (30) is supported on an annular shoulder (24'), which is present in the handle body (20').

7. The medical instrument (1) according to claim 6,
**characterised in that**
the annular shoulder (24') is provided by a proximal end of a guide sleeve (24), which is arranged in the handle body (20') to guide the sliding sleeve (22).

8. The medical instrument (1) according to at least any one of claims 3 to 7,
**characterised in that**
the optical waveguide (2) protrudes beyond the proximal end of the inner tube (4) and extends out of the handle (20) through a proximal axial end section (27) of the handle (20), wherein the proximal axial end section (27) has an annular sealing lip (28), which bears sealingly around the optical waveguide (2), wherein the annular sealing lip (28) is preferably radially adjustable or has a radially adjustable opening cross section, respectively.

9. The medical instrument (1) according to at least any one of claims 1 to 8,
**characterised in that**
the optical waveguide (2) is arranged in the inner tube (4) in an axially displaceable manner, wherein the handle (20) preferably has a second actuating element (40), which comprises a displacing device or is connected thereto, which is operatively connected to the optical waveguide (2) and which provides an axial displacement of the optical waveguide (2),
and/or
the handle (20) has an insertion aid (29) comprising an insertion opening, which tapers to an inner cross section of the inner tube (4), for the optical waveguide (2) into the inner tube (4).

10. The medical instrument according to at least any one of claims 1 to 9,
**characterised in that**
the wire formation of the tool (10)
- is a wire loop (11), which is formed from a first wire section (11') and a second wire section (11"), or
- is a small rock collecting basket (10) of two or three wire loops (11, 12, 13), which are in each case formed from a first wire section (11', 12', 13') and a second wire section (11", 12", 13").

11. The medical instrument according to any one of claims 1 to 10,
**characterised in that**
the wire formation
- is made of nitinol and has a pretension for the open position of the tool (10), and/or
- is at least partially provided by flat wire.

12. The medical instrument according to at least any one of claims 1 to 11,
**characterised in that**
- the outer tube (3) is made of a heat-resistant plastic, and/or
- the inner tube (4) has a fibre reinforcement comprising a friction-reducing plastic coating, wherein the fibre reinforcement is preferably a metal fibre reinforcement.

13. The medical instrument according to claim 12,
**characterised in that**
- the fastening of the proximal end (11b, 12b, 13b) of the at least one second wire section (11", 12", 13") to the outer tube (3) is provided by means of adhesion, smelting, and/or an additional heat-shrinkable tube section, which extends over a connecting section at the end of the outer tube (3) and the proximal end (11b, 12b, 13b) of the at least one second wire section (11", 12", 13"),
and/or
- the fastening of the proximal end (11a, 12a, 13a) of the at least one first wire section (11', 12', 13') to the inner tube (4)
a) is provided by means of adhesion, smelting, and/or an additional heat-shrinkable tube section, which extends over a connecting section at the end of the inner tube (4) and the proximal end (11a, 12a, 13a) of the at least one first wire section (11', 12', 13'), or
b) is provided by means of a frictional and/or material bonded connection of the proximal end (11a, 12a, 13a) of the at least one first wire section (11', 12', 13') to fibre ends of the fibre reinforcement, which are present at the distal end of the inner tube (4), or
c) is provided by means of an integral connection of the at least one first wire section (11', 12' 13') to the metal fibres of the fibre textile reinforcement of the inner tube (4), wherein the at least one first wire section (11', 12', 13') is formed from at least one portion of the metal fibre ends of the fibre textile reinforcement of the inner tube (4).

14. A method for producing a medical instrument (1) according to at least any one of claims 1 to 13,
**comprising the steps of**
- forming a tool (10) of at least two wire sections (11', 11", 12', 12", 13', 13") into a wire formation and providing a catheter assembly of an outer tube (3) and an inner tube (4), which is arranged coaxially thereto and which is movable relative thereto,
- fastening a proximal end (11a, 12a, 13a) of at least one first wire section (11', 12', 13') to the distal end of the inner tube (4) and
- fastening a proximal end (11b, 12b, 13b) of at least one second wire section (11", 12", 13") to the distal end of the outer tube (3), so that the tool (10) can be actuated to open and close by means of the relative movement between the outer tube (3) and the inner tube (4) without guide wire, and
- coaxially inserting the optical waveguide (2) into and through the inner tube (4), so that an exit end (2') of the optical waveguide (2) opens coaxially into a space, which is limited by the tool (10).

15. The method according to claim 14,
**comprising the steps of**
prior to the insertion of the optical waveguide (2),
- providing the optical waveguide (2) without buffering or removing the buffering from the optical waveguide (2), and/or
- providing a handle (20) comprising a handle body (20) and at least one actuating element (21) for actuating the tool (10), and
- connecting a proximal end section of the outer tube (3) to the actuating element (21), and
- storing a proximal end section of the inner tube (4), which protrudes in the handle (20) beyond the proximal end of the outer tube (3), in the handle (20), so that the outer tube (3) is axially displaceable with respect to the inner tube (4) in order to actuate the tool (10) by means of the actuating element (21).

## Revendications

1. Instrument médical (1) avec un outil (10), un ensemble cathéter et une poignée (20) permettant d'actionner l'outil (10) qui est une structure en fil métallique avec au moins deux parties de fil métallique (11', 11", 12', 12", 13', 13") et est disposé à une extrémité distale de l'ensemble cathéter, lequel est constitué d'un flexible extérieur (3) et d'un flexible intérieur (4) qui est disposé coaxialement par rapport à celui-ci, le flexible extérieur (3) et le flexible intérieur (4) étant mobiles l'un par rapport à l'autre, et l'instrument médical (1) présentant en outre un guide d'onde optique (2) qui s'étend à travers le flexible intérieur (4) et dont l'extrémité de sortie (2') aboutit coaxialement dans un espace limité par l'outil (10),
et l'instrument médical (1) ne présentant aucun fil de guidage pour l'actionnement de l'outil (10),
**caractérisé en ce que**
au moins une première partie de fil métallique (11', 12', 13') avec une extrémité proximale (11a, 12a, 13a) est fixée à l'extrémité distale du flexible intérieur (4) et au moins une deuxième partie de fil métallique (11", 12", 13") avec une extrémité proximale (11b, 12b, 13b) est fixée à l'extrémité distale du flexible extérieur (3),
et
**en ce que** l'outil (10) peut être actionné pour l'ouverture et la fermeture par le mouvement relatif entre le flexible extérieur (3) et le flexible intérieur (4).

2. Instrument médical (1) conformément à la revendication 1,
**caractérisé en ce que**
le guide d'onde optique (2) est une fibre laser (2) qui est conçue pour guider le rayonnement laser d'un laser au thulium, la fibre laser (2) ne présentant aucune zone tampon.

3. Instrument médical (1) conformément à la revendication 1 ou 2,
**caractérisé en ce que**
la poignée (20) présente un corps de poignée (20') et au moins un élément d'actionnement (21) pour actionner l'outil (10),
- une partie finale proximale du flexible extérieur (3) étant reliée à l'élément d'actionnement (21), et une partie finale proximale du flexible intérieur (4), laquelle dépasse de l'extrémité proximale du flexible extérieur (3) dans la poignée (20), étant montée dans la poignée (20), de façon à ce que le flexible extérieur (3) puisse coulisser axialement par rapport au flexible intérieur (4) au moyen de l'élément d'actionnement (21) pour l'actionnement de l'outil (10) ou
- une partie finale proximale du flexible extérieur (3) étant montée dans la pognée (20) et une partie finale proximale du flexible intérieur (4), laquelle dépasse de l'extrémité proximale du flexible extérieur (3) dans la poignée (20), étant reliée à l'élément d'actionnement (21), de façon à ce que le flexible intérieur (4) puisse coulisser axialement par rapport au flexible extérieur (3) au moyen de l'élément d'actionnement (21) pour l'actionnement de l'outil (10).

4. Instrument médical (1) conformément à la revendication 3,
**caractérisé en ce que**
l'élément d'actionnement (21) est une glissière de poignée (21) qui est coulissante dans le sens axial contre le corps de poignée (20') par rapport au mouvement du flexible extérieur (3)
et/ou
la liaison de l'élément d'actionnement (21) est assurée avec la partie finale proximale du flexible extérieur (3) par un manchon coulissant (22) qui est relié à l'élément d'actionnement (21) et dans lequel est monté un manchon d'assemblage (23) dans lequel est fixé le flexible extérieur (3).

5. Instrument médical (1) conformément à la revendication 3 ou 4,
**caractérisé en ce que**
le montage de la partie finale proximale du flexible intérieur (4) est assuré par un manchon de fixation (25) dans la poignée (20), dans lequel le flexible intérieur (4) est fixé et repose, avec son extrémité distale, sur un dispositif support qui est prévu entre l'extrémité proximale du flexible extérieur (3) et le manchon de fixation (25) dans le corps de poignée (20') ou dans un embout du corps de poignée (26), qui est relié à une extrémité proximale du corps de poignée (20').

6. Instrument médical (1) conformément à la revendication 5,
**caractérisé en ce que**
le dispositif support est un ressort (30), à l'extrémité proximale duquel se trouve le manchon de fixation (25), l'extrémité distale du ressort (30) s'appuyant sur un épaulement annulaire (24') qui se trouve dans le corps de poignée (20').

7. Instrument médical (1) conformément à la revendication 6,
**caractérisé en ce que**
l'épaulement annulaire (24') est assuré par une extrémité proximale d'un manchon de guidage (24) qui est disposé dans le corps de poignée (20') pour le guidage du manchon coulissant (22).

8. Instrument médical (1) conformément au moins à l'une des revendications 3 à 7,
**caractérisé en ce que**
le guide d'onde optique (2) dépasse de l'extrémité proximale du flexible intérieur (4) et s'étend depuis la poignée (20) par une partie finale axiale proximale (27) de la poignée (20), la partie finale axiale proximale (27) présentant un joint circulaire (28) qui assure l'étanchéité autour du guide d'onde optique (2), le joint circulaire (28) étant préférablement réglable dans le sens radial et/ou présentant une section d'ouverture réglable dans le sens radial.

9. Instrument médical (1) conformément au moins à l'une des revendications 1 à 8,
**caractérisé en ce que**
le guide d'onde optique (2) est coulissant axialement dans le flexible intérieur (4), la poignée (20) présentant préférablement un deuxième élément d'actionnement (40) qui comprend un dispositif de déplacement ou est relié à celui-ci, lequel est en liaison active avec le guide d'onde optique (2) et assure un déplacement axial du guide d'onde optique (2)
et/ou
la poignée (20) présente une aide à l'introduction (29) avec une ouverture d'introduction pour le guide d'onde optique (2) dans le flexible intérieur (4), laquelle se rétrécit en direction d'une section transversale intérieure du flexible intérieur (4).

10. Instrument médical conformément au moins à l'une des revendications 1 à 9,
**caractérisé en ce que**
la structure en fil métallique de l'outil (10) est
- une boucle en fil métallique (11) qui est constituée d'une première partie de fil métallique (11') et d'une deuxième partie de fil métallique (11") ou
- un panier collecteur (10) composé de deux ou trois boucles en fil métallique (11, 12, 13), lesquelles sont respectivement constituées d'une première partie de fil métallique (11', 12', 13') et d'une deuxième partie de fil métallique (11", 12", 13") .

11. Instrument médical conformément au moins à l'une des revendications 1 à 10,
**caractérisé en ce que**
la structure en fil métallique est
- en Nitinol et présente une précontrainte pour la position ouverte de l'outil (10) et/ou
- est assurée au moins en partie par un fil métallique plat.

12. Instrument médical conformément au moins à l'une des revendications 1 à 11,
**caractérisé en ce que**
- le flexible extérieur (3) est fabriqué à partir d'une matière plastique résistant à la chaleur
et/ou
- le flexible intérieur (4) présente une armature en fibres avec un revêtement en plastique diminuant le frottement, l'armature en fibres étant préférablement une armature en fibres métalliques.

13. Instrument médical conformément à la revendication 12,
**caractérisé en ce que**
- la fixation de l'extrémité proximale (11b, 12b, 13b) au moins d'une deuxième partie de fil métallique (11", 12", 13") sur le flexible extérieur (3) est assurée par collage, fusion et/ou une partie de gaine rétractable supplémentaire qui s'étend par une partie de liaison à l'extrémité du flexible extérieur (3) et l'extrémité proximale (11b, 12b, 13b) au moins d'une deuxième partie de fil métallique (11", 12", 13") et/ou
- la fixation de l'extrémité proximale (11a, 12a, 13a) au moins d'une première partie de fil métallique (11', 12', 13') sur le flexible intérieur (4) est assurée
a) par collage, fusion et/ou une partie de gaine rétractable supplémentaire qui s'étend par une partie de liaison à l'extrémité du flexible intérieur (4) et l'extrémité proximale (11a, 12a, 13a) au moins d'une première partie de fil métallique (11', 12', 13') ou
b) par une liaison par friction et/ou conjugaison de matière de l'extrémité proximale (11a, 12a, 13a) au moins d'une première partie de fil métallique (11', 12', 13') avec des extrémités de fibres de l'armature en fibres, qui se trouvent à l'extrémité distale du flexible intérieur (4) ou
c) par une liaison intégrale au moins d'une première partie de fil métallique (11', 12', 13') avec les fibres métalliques de l'armature en fibres textiles du flexible intérieur (4), au moins une première partie de fil métallique (11', 12', 13') étant formée au moins d'une partie des extrémités de fibres métalliques de l'armature en fibres textiles du flexible intérieur (4).

14. Procédé de fabrication d'un instrument médical (1) conformément au moins à l'une des revendications 1 à 13,
**comprenant les étapes**
- formation d'un outil (10) à partir au moins de deux parties de fil métallique (11', 11", 12', 12", 13', 13") en une structure en fil métallique et mise à disposition d'un ensemble cathéter composé d'un flexible extérieur (3) et d'un flexible intérieur (4) disposé coaxialement et de façon mobile par rapport à celui-ci,
- fixation d'une extrémité proximale (11a, 12a, 13a) au moins d'une première partie de fil métallique (11', 12', 13') à l'extrémité distale du flexible intérieur (4) et
- fixation d'une extrémité proximale (11b, 12b, 13b) au moins d'une deuxième partie de fil métallique (11", 12", 13") à l'extrémité distale du flexible extérieur (3), de façon à ce que l'outil (10) puisse être actionné sans fil de guidage pour l'ouverture et la fermeture par le mouvement relatif entre le flexible extérieur (3) et le flexible intérieur (4) et
- introduction coaxiale du guide d'onde optique (2) dans et à travers le flexible intérieur (4), de façon à ce qu'une extrémité de sortie (2') du guide d'onde optique (2) aboutisse coaxialement dans un espace limité par l'outil (10).

15. Procédé conformément à la revendication 14,
**comprenant les étapes**
avant l'introduction du guide d'onde optique (2),
- mise à disposition du guide d'onde optique (2) sans zone tampon ou retrait de la zone tampon du guide d'onde optique (2) et/ou
- mise à disposition d'une poignée (20) avec un corps de poignée (20) et au moins un élément d'actionnement (21) pour actionner l'outil (10) et
- liaison d'une partie finale proximale du flexible extérieur (3) avec l'élément d'actionnement (21) et
- montage d'une partie finale proximale du flexible intérieur (4) dans la poignée (20), laquelle dépasse de l'extrémité proximale du flexible extérieur (3) dans la poignée (20), de façon à ce que le flexible extérieur (3) puisse coulisser axialement par rapport au flexible intérieur (4) au moyen de l'élément d'actionnement (21) pour l'actionnement de l'outil (10).
